# FASCICULE DE BREVET EUROPEEN

(11) **EP 2 892 504 B1**
(45) Date de publication et mention de la délivrance du brevet: **01.02.2017**
(21) Numéro de dépôt: 13767046.9
(22) Date de dépôt: 10.09.2013
(51) Int. Cl.: A61K 9/00, A61K 39/00, A61K 47/16, A61K 9/08

(54) **SOLUTION AQUEUSE DE PROTÉINE À CONCENTRATION ÉLEVÉE DE VISCOSITÉ RÉDUITE**
HOCHKONZENTRIERTE WÄSSRIGE PROTEINLÖSUNG MIT REDUZIERTER VISKOSITÄT
HIGH CONCENTRATION AQUEOUS PROTEIN SOLUTION HAVING REDUCED VISCOSITY

(30) Priorité: 10.09.2012 FR 1258494; 10.09.2012 US 201261699053 P
(43) Date de publication de la demande: 15.07.2015
(73) Titulaire: Adocia, 69003 Lyon (FR)
(72) Inventeur: SOULA, Olivier, F-69330 Meyzieu (FR)
(74) Mandataire: Tripoz, Inès
(86) Numéro de dépôt international: PCT/FR2013/052076
(87) Numéro de publication internationale: WO 2014/037680

(56) Documents cités:
- WO-A1-2010/132047
- WO-A2-2011/121560
- US-A1- 2002 045 571
- Anonymous: "An introduction to clinical pharmaceutics", 2010, Pharmaceutical Press, Great Britain, XP002696437, page 73, alinéa 2

## Description

Les anticorps monoclonaux (identifiés ci-après sous l'appellation mAbs) sont une classe de protéines thérapeutiques en pleine expansion pour traiter des pathologies graves, comme certains cancers, maladies infectieuses et maladies auto-immunes. Actuellement, plus de 30 mAbs ont été approuvés aux Etats-Unis et en Europe, et environ 20% des protéines thérapeutiques actuellement en développement sont des anticorps.

Les anticorps peuvent être administrés par voie parentérale, telle que l'injection intraveineuse (IV), sous-cutanée (SC) ou intramusculaire (IM). Les voies SC et IM permettent de réduire le coût des traitements et améliorent le confort des patients.

Pour les injections SC et IM, le faible volume pouvant être injecté (0,5-2 mL) impose le développement de formulations concentrées en anticorps puisque les doses requises sont classiquement de 100 mg à 1 g pour atteindre un effet thérapeutique. A des concentrations supérieures à 100 mg/mL, les solutions aqueuses de mAbs sont souvent instables et visqueuses rendant difficiles les étapes de fabrication (étapes de purification, concentration et de répartition) comme cela est décrit dans la publication de Shire, Current Opinion in Biotechnology, 2009, 20, 708-714.

Le problème de la viscosité peut également être rencontré pour les formulations de nanobodies et de protéines de fusion.

La viscosité des formulations entraîne des problèmes d'injectabilité et rend les administrations douloureuses pour le patient comme cela est décrit dans la publication de Cilurzo et al., AAPS PharmSciTech, 2011, 12(2), 604-9.

Cette viscosité des formulations concentrées en protéines devient donc un véritable challenge comme cela est décrit dans la publication de Adler, American Pharmaceutical Review, February 2012, 15(1).

Même si les facteurs régissant ces augmentations de viscosité sont assez mal décrits et identifiés, de nombreuses publications montrent que l'augmentation de viscosité des solutions aqueuses d'anticorps à haute concentration résulte d'au moins deux facteurs : des effets stériques (exclusion de volume) et des interactions protéines-protéines comme cela est décrit dans la publication de Saluja & Katonia, Int J Pharm, 2008, 1-15.

Cependant, il s'agit du seul facteur susceptible d'être modifié par le formulateur. En effet, dans la plupart des cas, les autres facteurs que sont les doses et les calibres des aiguilles ne peuvent pas être modifiés. Ainsi, le besoin d'identifier des excipients réducteurs de viscosité susceptibles d'abaisser la viscosité de solutions à haute concentration de protéines est par conséquent devenu crucial et stratégique dans l'industrie pharmaceutique.

L'utilisation d'un sel comme le NaCl a été décrite notamment dans la publication de Shire & al., J Pharm Sci 2004, 1390-402 ou le brevet US7,666,413 comme permettant d'abaisser la viscosité des solutions aqueuses de mAb concentrées. Mais cette solution n'est cependant pas universelle et la viscosité de nombreuses formulations de mAb n'est pas réduite ou pas suffisamment réduite par l'addition d'un sel comme le NaCl, comme cela est confirmé dans la publication de Guo & al., Pharm Res, 2012, 29(11), 3102-9.

Des acides aminés (brevet US7,666,413) et des dérivés d'acides aminés (brevet WO2011139718) ont également été décrits comme permettant de réduire la viscosité de formulations contenant une protéine active de type mAb. En particulier, le sel chlorhydrate d'arginine, composé couramment utilisé dans les procédés de purification et de formulation des protéines, voir par exemple la publication de Frokjaer S, Nat Rev Drug Discov,2005, 4(4), 298-306, peut être considéré comme une des références pour apprécier les abaissements de viscosité.

La viscosité des formulations mAbs peut dans certains cas augmenter du fait de la tendance qu'ont les mAbs à s'agréger à hautes concentrations comme le décrit la publication de Florence, *Pharmaceutical Press,* 2010. Dans d'autres cas, les formulations mAbs à hautes concentrations peuvent être visqueuses sans que les mAbs s'agrègent. Dans d'autres cas encore, les mAbs peuvent s'agréger sans que ces formulations à hautes concentrations en mAbs soient visqueuses. Dans encore d'autres cas, les formulations à hautes concentrations en mAbs sous forme agrégée ou cristallisée peuvent présenter une viscosité inférieure à des formulations à hautes concentrations en mAbs équivalentes mais qui sont sous forme non agrégée ou non cristallisée. Ainsi, augmentation de viscosité et augmentation de l'agrégation ne sont pas systématiquement corrélées. De la même manière, la réduction de viscosité et la réduction de l'agrégation ne sont pas toujours corrélées.

La demande de brevet WO2011/121560 décrit un procédé de stabilisation d'un anticorps dans un environnement liquide comprenant la modulation de l'agrégation dudit anticorps par liaison ou masquage d'une lysine spécifique de la région Fc de l'anticorps (résidu Lys445). Cette demande exemplifie plus particulièrement des compositions comprenant du bevacizumab et un nucléotide, i.e. adénosine 5'-monophosphate (AMP), guanosine 5'-monophosphate (GMP) ou adénosine 5'-triphosphate (ATP). Le(s) phosphate(s) des nucléotides interagisse(nt) avec le résidu Lys445 de la région Fc du bevacizumab, permettant ainsi de stabiliser le bevacizumab en diminuant l'agrégation dudit anticorps. Tous les composés décrits ou revendiqués portent une chaîne chargée négativement, de préférence phosphate, qui va permettre cette interaction avec le résidu Lys445 de la région Fc des anticorps. La demande de brevet WO2011/121560 n'évoque pas la réduction de viscosité. De plus il a été démontré par la demanderesse que l'ATP, qui stabilise une formulation commerciale de bevacizumab, ne permet pas de réduire la viscosité ni de bevacizumab ni d'infliximab. L'ATP augmente au contraire la viscosité de bevacizumab (+ 14% d'augmentation).

De façon surprenante, la demanderesse a identifié des molécules naturelles de la famille des nucléosides capables d'abaisser la viscosité d'une solution aqueuse difficilement injectable d'au moins une protéine comportant au moins un fragment d'anticorps.

Selon la présente invention, les termes « agent réducteur de viscosité » et « composé réducteur de viscosité » peuvent être employés de manière interchangeable.

On entend par « nucléosides », des glycosylamines naturelles constituées d'une base liée à un ribose ou un désoxyribose via une liaison glycosidique. Parmi les nucléosides, on retrouve notamment la cytidine, l'uridine, la thymidine ou encore l'inosine. Dans les cellules, les nucléosides peuvent être phosphorylés par des kinases spécifiques, permettant la formation des nucléotides qui sont les éléments constitutifs de l'ADN et de l'ARN.

La demande de brevet WO2010/132047 décrit une composition gel guanosine/GMP pour la délivrance d'anticorps, d'acides nucléiques et de particules. La très mauvaise solubilité de la guanosine ne permet néanmoins pas son utilisation dans une composition à viscosité réduite.

La présente invention concerne une méthode pour réduire la viscosité consistant à ajouter, à la solution aqueuse difficilement injectable d'au moins une protéine comportant au moins un fragment d'anticorps, au moins un agent réducteur de viscosité soluble dans l'eau choisi dans le groupe constitué par la cytidine, la 2'-deoxycytidine, l'uridine, la 2'-deoxyuridine, la thymidine, et la ribothymidine, seule ou en mélange.

Dans un mode de réalisation, le composé réducteur de viscosité est choisi dans le groupe constitué par la cytidine, l'uridine et la ribothymidine, seule ou en mélange.

Dans un mode de réalisation, le composé réducteur de viscosité est choisi dans le groupe constitué par la 2'-deoxycytidine, la 2'-deoxyuridine et la thymidine, seule ou en mélange.

Dans un mode de réalisation, le composé réducteur de viscosité est choisi dans le groupe constitué par la cytidine, et la 2'-deoxycytidine, seule ou en mélange.

Dans un mode de réalisation, le composé réducteur de viscosité est choisi dans le groupe constitué par l'uridine, et la 2'-deoxyuridine, seule ou en mélange.

Dans un mode de réalisation, le composé réducteur de viscosité est choisi dans le groupe constitué par la thymidine et la ribothymidine, seule ou en mélange.

Dans un mode de réalisation, le composé réducteur de viscosité est choisi dans le groupe constitué de la cytidine, la 2'-deoxycytidine et l'uridine seule ou en mélange.

Dans un mode de réalisation, le composé réducteur de viscosité est choisi dans le groupe constitué par la 2'-deoxyuridine, la thymidine et la ribothymidine, seule ou en mélange.

Dans un mode de réalisation, le composé réducteur de viscosité est la cytidine.

Dans un mode de réalisation, le composé réducteur de viscosité est la 2'-deoxycytidine.

Dans un mode de réalisation, le composé réducteur de viscosité est l'uridine.

Dans un mode de réalisation, le composé réducteur de viscosité est la 2'-deoxyuridine.

Dans un mode de réalisation, le composé réducteur de viscosité est la thymidine.

Dans un mode de réalisation, le composé réducteur de viscosité est la ribothymidine.

Toutes ces molécules sont présentes dans les tissus biologiques.

Par « soluble dans l'eau », on entend une solubilité minimum de 50 mM, voir une solubilité de 250 mM, à un pH compris entre 5 et 8, et à 25°C.

On entend par « agent réducteur de viscosité » ou « composé réducteur de viscosité », un composé capable, selon l'invention, de réduire la viscosité d'une solution aqueuse difficilement injectable d'au moins une protéine comprenant au moins un fragment d'anticorps, d'une valeur d'au moins 10 %. Les techniques pour mesurer la viscosité sont connues de l'homme du métier. En particulier, la viscosité est mesurée selon la technique décrite à l'exemple 2.

Dans un mode de réalisation, la viscosité est abaissée d'une valeur comprise entre 10 et 90%.

Dans un mode de réalisation, la viscosité est abaissée d'une valeur comprise entre 20 et 90%.

Dans un mode de réalisation, la viscosité est abaissée d'une valeur comprise entre 30 et 85%.

Dans un mode de réalisation, la viscosité est abaissée d'une valeur comprise entre 35 et 80%.

Dans un mode de réalisation, la viscosité est abaissée d'une valeur comprise entre 35 et 77%.

Dans un mode de réalisation, la viscosité est abaissée d'une valeur comprise entre 60 et 90%.

Dans un mode de réalisation, la viscosité est abaissée d'une valeur comprise entre 60 et 80%.

Dans un mode de réalisation, la viscosité est abaissée d'une valeur d'au moins 15%.

On entend par « solution aqueuse », une solution dans laquelle l'eau est le solvant principal. Une solution aqueuse peut en outre comprendre un tampon, un agent permettant de contrôler l'osmolalité, un tensio-actif, un agent cryoprotectant, un agent lyoprotecteur et tout excipent pharmaceutiquement acceptable, nécessaire à la préparation d'une composition pharmaceutique.

On entend par « solution aqueuse difficilement injectable » d'au moins une protéine comprenant au moins un fragment d'anticorps » une solution aqueuse d'au moins une protéine comprenant au moins un fragment d'anticorps dont la viscosité est au moins de 15 cP. Il est généralement admis qu'une telle solution dont la viscosité est au moins de 15 cP est difficilement injectable par voie sous-cutanée à l'aide d'une aiguille d'un calibre d'au moins 29 G. La demanderesse a retenu comme limite le calibre d'au moins 29 G car ce dernier est le calibre supérieur admissible pour que le confort d'injection ne soit pas trop dégradé.

On entend par « protéine comprenant au moins un fragment d'anticorps » une protéine choisie parmi les anticorps monoclonaux (mAbs), les anticorps polyclonaux, les protéines de fusion, les nanobodies, les anticorps bispécifiques et les anticorps couplés à des principes actifs cytotoxiques (ADC).

On entend par « anticorps monoclonal » un « anticorps complet », un « fragment d'anticorps » ou un « dérivé d'anticorps » qui possède une spécificité identique et unique i.e. qui ne reconnait qu'un seul type d'épitope sur un antigène donné.

Dans un mode de réalisation, la protéine comportant au moins un fragment d'anticorps est un anticorps monoclonal.

Selon la présente invention, un anticorps peut aussi être appelé immunoglobuline.

On entend par « anticorps complet » un anticorps composé de deux chaînes lourdes identiques (" CH ") et de deux chaînes légères identiques (" CL ") qui sont liées par un pont disulfure. Chaque chaîne est constituée, en position N-terminale, d'une région (ou domaine) variable (codée par les gènes réarrangés V-J pour les chaînes légères et V-D-J pour les chaînes lourdes) spécifique de l'antigène contre lequel l'anticorps est dirigé, et en position C-terminale, d'une région constante, constituée d'un seul domaine CL pour les chaînes légères ou de plusieurs domaines pour les chaînes lourdes. Chaque région variable comprend trois segments appelés " régions déterminant la complémentarité " (" CDRs ") ou " régions hypervariables ", qui sont principalement responsables de la liaison à l'épitope d'un antigène. Les deux chaînes lourdes (H, Heavy) et les deux chaînes légères (L, Light) sont identiques entre elles. La chaîne légère est composée de 2 domaines, un domaine variable V et un domaine constant C, repliés indépendamment l'un de l'autre dans l'espace. On les appelle VL et CL. La chaîne lourde comporte également un domaine V noté VH et 3 ou 4 domaines C noté de CH1 à CH4. Chaque domaine comprend environ 110 acides aminés et est structuré de manière comparable. Les 2 chaînes lourdes sont liées par des ponts disulfures et chaque chaîne lourde est liée à une chaîne légère par un pont disulfure également. La région qui détermine la spécificité de l'anticorps pour l'antigène est portée par les parties variables, alors que les parties constantes peuvent interagir avec les récepteurs Fc des cellules effectrices ou des molécules comme le complément pour médier différentes propriétés fonctionnelles. Le terme " VH " fait référence aux régions variables d'une chaîne lourde d'immunoglobuline d'un anticorps, incluant les chaînes lourdes d'un fragment Fv, scFv, dsFv, Fab, Fab' ou F(ab)'. Le terme " VL " fait référence aux régions variables d'une chaîne légère d'immunoglobuline d'un anticorps, incluant les chaînes légères d'un fragment Fv, scFv, dsFv, Fab, Fab' ou F(ab)'. Par " régions CDR ou CDRs ", on entend désigner les régions hypervariables des chaînes lourdes et légères des immunoglobulines comme définies par Kabat et al. (Kabat et al., Sequences of proteins of immunological interest, 5th Ed., US Department of Health and Human Services, NIH, 1991 and later editions, XV). Il existe 3 CDRs de chaîne lourde et 3 CDRs de chaîne légère. Le terme CDR ou CDRs est utilisé ici pour désigner suivant les cas, l'une de ces régions ou plusieurs, voire l'ensemble, de ces régions qui contiennent la majorité des résidus d'acides aminés responsables de la liaison affine de l'anticorps pour l'antigène ou l'épitope qu'il reconnaît. Les régions les plus conservées des domaines variables sont appelées régions ou séquences FR pour " framework " ou régions " charpentes" au nombre de 4 (FR 1 à FR4).

Les anticorps sont subdivisés en 5 classes ou isotypes : IgG, IgA, IgM, IgE et IgD selon la structure des domaines constants des chaînes lourdes, i.e. respectivement chaînes γ, α, µ, ε et δ.

Les classes des IgG et des IgA sont par ailleurs subdivisées en sous-classes selon notamment la taille des régions charnières ainsi que le nombre et la position de ponts disulfures entre chaînes lourdes.

La classe des IgG est subdivisée en 4 sous-classes i.e. IgG1, IgG2, IgG3 et IgG4.

La classe des IgA est quant à elle subdivisée en 2 sous-classes i.e. IgA1 et IgA2.

Dans un mode de réalisation, l'anticorps est un IgG.

Dans un mode de réalisation, l'anticorps est un IgA.

Dans un mode de réalisation, l'anticorps est un IgM.

Dans un mode de réalisation, l'anticorps est un IgE.

Dans un mode de réalisation, l'anticorps est un IgD.

Dans un mode de réalisation, l'anticorps est un IgG1.

Dans un mode de réalisation, l'anticorps est un IgG2.

Dans un mode de réalisation, l'anticorps est un IgG3.

Dans un mode de réalisation, l'anticorps est un IgG4.

Dans un mode de réalisation, l'anticorps est un IgA1.

Dans un mode de réalisation, l'anticorps est un IgA2.

On entend par « fragment d'anticorps » tout fragment fonctionnel d'anticorps e.g. Fab (Fragment antigen binding), Fv, scFv (single chain Fv), Fc (Fragment cristallisable), F(ab') 2, Fab', scFv-Fc, des polypeptides synthétiques contenant les séquences d'un ou de CDRs, qui possèdent généralement la même spécificité de fixation que l'anticorps dont ils sont issus.

Selon la présente invention, des fragments d'anticorps de l'invention peuvent être obtenus à partir des anticorps par des méthodes telles que la digestion par des enzymes, comme la pepsine ou la papaïne et/ou par clivage des ponts disulfures par réduction chimique. La digestion enzymatique des anticorps par la papaïne génère 2 fragments identiques, qu'on appelle « fragment Fab (Fragment Antigen Binding), et un fragment Fc (fragment cristallisable). Le fragment Fc est le support des fonctions effectrices des immunoglobulines. Par digestion à la pepsine, un fragment F (ab') 2 est généré, où les deux fragments Fab restent liés par deux ponts disulfure, et le fragment Fc est scindé en plusieurs peptides. Le fragment F (ab') 2 est formé de deux fragments Fab', liés par des ponts disulfure intercaténaires pour former un F(ab')2.

Ainsi, l' « anticorps monoclonal » selon l'invention pouvant avantageusement contenir un ou plusieurs de ces fragments, toutes les combinaisons entre les fragments précédemment cités font partie de l'invention.

On entend par « dérivé d'anticorps » tout anticorps, cet anticorps pouvant comprendre une ou plusieurs mutations, substitutions, délétions et/ou additions d'un ou plusieurs résidus d'acides aminés. Un tel ajout, substitution ou délétion peut être localisé à n'importe quelle position dans la molécule. Dans le cas où plusieurs acides aminés ont été ajoutés, substitués ou délétés, toute combinaison d'ajout, de substitution ou de délétion peut être considérée, à condition que l'anticorps résultant présente toujours au moins les propriétés avantageuses de l'anticorps de l'invention.

Selon l'invention, l'« anticorps monoclonal » peut avantageusement être un « anticorps chimérique » ou un «anticorps humanisé ». Par « anticorps chimérique » on entend un anticorps dont les régions variables des chaînes légères et lourdes, ou au moins un domaine ou fragment de ces régions, appartiennent à une espèce différente de l'espèce à laquelle appartiennent les régions constantes des chaînes légères et des chaînes lourdes. Par « anticorps humanisé » on entend un anticorps qui contient principalement des séquences immunoglobuline humaines. Ce terme fait généralement référence à une immunoglobuline non humaine qui a été modifiées par incorporation de séquences humaines ou de résidus trouvés dans des séquences humaines.

Les anticorps selon l'invention peuvent être constitués en utilisant les techniques standard de l'ADN recombinant, bien connues de l'homme du métier, par exemple en utilisant les techniques de construction des anticorps « chimériques » décrites par exemple dans Morrison et al., Proc. Natl. Acad. Sci. U.S.A., 1984, 81(21), 6851-55, où la technologie de l'ADN recombinant est utilisée pour remplacer la région constante d'une chaîne lourde et/ou la région constante d'une chaîne légère d'un anticorps provenant d'un mammifère non-humain avec les régions correspondantes d'une immunoglobuline humaine. De tels anticorps et leur mode de préparation ont également été décrits dans la demande de brevet EP 173 494, dans le document Neuberger, M.S. et al., Nature 312 (5995) : 604-8 (1985), ainsi que dans le document EP 125 023 par exemple. Des méthodes pour générer des anticorps chimériques sont largement disponibles pour l'homme du métier. Par exemple, les chaînes lourdes et légères de l'anticorps peuvent être exprimées séparément en utilisant un vecteur pour chaque chaîne, ou bien être intégrées dans un seul vecteur.

A titre d'exemple on citera parmi les anticorps monoclonaux commercialisés les anticorps monoclonaux suivants : le Muromonab-CD3 (commercialisé sous le nom de Orthoclone Okt3^{®}), l'Abciximab (commercialisé sous le nom de Reopro^{®}), le Rituximab (commercialisé sous les noms de MabThera^{®} et Rituxan^{®}), le Basiliximab (commercialisé sous le nom de Simulect^{®}), le Daclizumab (commercialisé sous le nom de Zenapax^{®}), le Palivizumab (commercialisé sous le nom de Synagis^{®}), l'Infliximab (commercialisé sous le nom de Remicade^{®}), le Trastuzumab (commercialisé sous le nom de Herceptin^{®}), l'Alemtuzumab (commercialisé sous les noms de MabCampath^{®}, Campath-1H^{®}), l'Adalimumab (commercialisé sous le nom de Humira^{®}), le Tositumomab-I131 (commercialisé sous le nom de Bexxar^{®}), l'Efalizumab (commercialisé sous le nom de Raptiva^{®}), le Cetuximab (commercialisé sous le nom de Erbitux^{®}), l'Ibritumomab tiuxetan (commercialisé sous le nom de Zevalin^{®}), l'Omalizumab (commercialisé sous le nom de Xolair^{®}), le Bevacizumab (commercialisé sous le nom de Avastin^{®}), le Natalizumab (commercialisé sous le nom de Tysabri^{®}), le Ranibizumab (commercialisé sous le nom de Lucentis^{®}), le Panitumumab (commercialisé sous le nom de Vectibix^{®}), l'Eculizumab (commercialisé sous le nom de Soliris^{®}), le Certolizumab pegol (commercialisé sous le nom de Cimzia^{®}), le Golimumab (commercialisé sous le nom de Simponi^{®}), le Canakinumab (commercialisé sous le nom de Ilaris^{®}), le Catumaxomab (commercialisé sous le nom de Removab^{®}), l'Ustekinumab (commercialisé sous le nom de Stelara^{®}), le Tocilizumab (commercialisé sous les noms de RoActemra^{®}, et Actemra^{®}), l'Ofatumumab (commercialisé sous le nom de Arzerra^{®}), le Denosumab (commercialisé sous le nom de Prolia^{®}), le Belimumab (commercialisé sous le nom de Benlysta^{®}), le Raxibacumab (pas encore commercialisé), l'Ipilimumab (commercialisé sous le nom de Yervoy^{®})et le Pertuzumab (commercialisé sous le nom de Perjeta^{®}).

On entend par « anticorps polyclonal » un mélange d'« anticorps complets », un mélange de « fragments d'anticorps » ou un mélange de « dérivés d'anticorps » reconnaissant différents types d'épitopes sur un antigène donné.

Dans un mode de réalisation, la protéine comportant au moins un fragment d'anticorps est un anticorps polyclonal.

On entend par « protéines de fusion » une construction qui renferme plusieurs protéines ou polypeptides d'origine différente. Cette protéine de fusion est codée par un acide nucléique obtenu par les technologies d'ADN recombinant bien connues de l'homme du métier. Selon la présente invention, la protéine de fusion est constituée par un fragment d'« anticorps monoclonal » tel que précédemment décrit et un fragment d'une protéique d'intérêt.

Dans un mode de réalisation, la protéine comportant au moins un fragment d'anticorps est une protéine de fusion.

A titre d'exemple on citera la protéine de fusion constituée par un fragment d'anticorps monoclonal qui est la région Fc d'une immunoglobuline IgG1 et un fragment d'une protéine d'intérêt qui est le domaine extra-cellulaire du récepteur de protéine CTLA-4 (Cytotoxic T-Lymphocyte Antigen 4), cette protéine de fusion i.e. abatacept, étant commercialisée sous le nom d'Orencia^{®}.

A titre d'exemple on citera également la protéine de fusion constituée par un fragment d'anticorps monoclonal qui est la région Fc d'une IgG1 et un fragment d'une protéine d'intérêt qui est la fraction P75 du récepteur soluble du TNF-alpha, cette protéine de fusion i.e. etanercept étant commmercialisée sous le nom Enbrei^{®}.

A titre d'exemple on citera également la protéine de fusion constituée par un fragment d'anticorps monoclonal qui est la région Fc d'une IgG1 et un fragment d'une protéine d'intérêt qui sont les portions extracellulaires de l'IL-1R1 (interleukin-1 receptor component) et de IL-1RAcP (IL-1 receptor accessory protein), cette protéine de fusion i.e. rilonacept étant commercialisée sous le nom de Arcalyst^{®}.

A titre d'exemple on citera également la protéine de fusion constituée par un fragment d'anticorps monoclonal qui sont les régions IgG1 charnière, C(H)2 et C(H)3, et un fragment d'une protéine d'intérêt qui est le domaine extracellulaire de LFA-3, cette protéine de fusion i.e. alefacept étant commercialisée sous le nom d'Amevive^{®}.

On entend par « nanobody » tout domaine variable unique de chaînes lourdes d'immunoglobuline. Les nanobodies sont plus largement décrits dans la publication C.Vincke and S.Muyldermans, in D. Saerens and S. Muyldermans (eds.) Single Domain Antibodies : Methods and Protocols, Methods in Molecular Biology, vol. 911, Springer Science+Business Media, LLC2012,15-26; et Wesolowski & al., Med Microbiol Immunol (2009), 198(3), 157-174.

Dans un mode de réalisation, la protéine comportant au moins un fragment d'anticorps est un nanobody.

On entend par « anticorps bispécifique » (aussi appelé anticorps bifonctionnel ou « diabody ») tout fragment d'immunoglobuline comprenant 2 sites de présentation à l'antigène. Les anticorps bifonctionnels sont plus largement décrits dans la publication Hollinger et al., Proc. Natl. Acad. Sci. USA 90(4): 6444-6448 (1993).

Dans un mode de réalisation, la protéine comportant au moins un fragment d'anticorps est un anticorps bispécifique.

On entend par « anticorps couplé à un principe actif cytotoxique » un « anticorps monoclonal » tel que précédemment décrit couplé à un principe actif cytotoxique.

A titre d'exemple de principe actif cytotoxique, on citera notamment vedotin.

Dans un mode de réalisation, la protéine comportant au moins un fragment d'anticorps est un anticorps couplé à un principe actif cytotoxique.

A titre d'exemple l'anticorps couplé à un principe actif cytotoxique est l'anticorps brentuximab couplé au principe actif cytotoxique vedotin. Cet anticorps couplé à ce principe actif cytotoxique est commercialisé sous le nom d'Adcetris^{®}.

L'invention concerne également une composition comprenant, en solution aqueuse, au moins une protéine comportant au moins un fragment d'anticorps, et au moins un agent réducteur de viscosité soluble dans l'eau, choisi dans le groupe constitué par la cytidine, la 2'-deoxycytidine, l'uridine, la 2'-deoxyuridine, la thymidine et la ribothymidine , seule ou en mélange.

L'invention concerne également une composition pharmaceutique pour l'administration par voie sous-cutanée (SC) comprenant en solution aqueuse, au moins une protéine comportant au moins un fragment d'anticorps, et au moins un agent réducteur de viscosité choisi dans le groupe constitué par la cytidine, la 2'-deoxycytidine, l'uridine, la 2'-deoxyuridine, la thymidine et la ribothymidine, seule ou en mélange.

L'invention concerne également une composition pharmaceutique pour l'administration par voie intraveineuse (IV), comprenant en solution aqueuse, au moins une protéine comportant au moins un fragment d'anticorps, et au moins un agent réducteur de viscosité choisi dans le groupe constitué par la cytidine, la 2'-deoxycytidine, l'uridine, la 2'-deoxyuridine, la thymidine et la ribothymidine, seule ou en mélange.

L'invention concerne également une composition pharmaceutique pour l'administration par voie intramusculaire (IM) comprenant en solution aqueuse, au moins une protéine comportant au moins un fragment d'anticorps, et au moins un agent réducteur de viscosité choisi dans le groupe constitué par la cytidine, la 2'-deoxycytidine, l'uridine, la 2'-deoxyuridine, la thymidine et la ribothymidine, seule ou en mélange.

Dans un mode de réalisation de la composition, le composé réducteur de viscosité est choisi dans le groupe constitué par la cytidine, l'uridine et la ribothymidine, seule ou en mélange.

Dans un mode de réalisation de la composition, le composé réducteur de viscosité est choisi dans le groupe constitué par la 2'-deoxycytidine, la 2'-deoxyuridine et la thymidine, seule ou en mélange.

Dans un mode de réalisation de la composition, le composé réducteur de viscosité est choisi dans le groupe constitué par la cytidine et la 2'-deoxycytidine, seule ou en mélange.

Dans un mode de réalisation de la composition, le composé réducteur de viscosité est choisi dans le groupe constitué par l'uridine et la 2'-deoxyuridine, seule ou en mélange.

Dans un mode de réalisation de la composition, le composé réducteur de viscosité est choisi dans le groupe constitué par la thymidine et la ribothymidine, seule ou en mélange.

Dans un mode de réalisation de la composition, le composé réducteur de viscosité est choisi dans le groupe constitué de la cytidine, la 2'-deoxycytidine et l'uridine, seule ou en mélange.

Dans un mode de réalisation de la composition le composé réducteur de viscosité est choisi dans le groupe constitué par la 2'-deoxyuridine, la thymidine et la ribothymidine, seule ou en mélange.

Dans un mode de réalisation de la composition, le composé réducteur de viscosité est la cytidine.

Dans un mode de réalisation de la composition, le composé réducteur de viscosité est la 2'-deoxycytidine.

Dans un mode de réalisation de la composition, le composé réducteur de viscosité est l'uridine.

Dans un mode de réalisation de la composition, le composé réducteur de viscosité est la 2'-deoxyuridine.

Dans un mode de réalisation de la composition, le composé réducteur de viscosité est la thymidine.

Dans un mode de réalisation de la composition, le composé réducteur de viscosité est la ribothymidine.

Une concentration exprimée en M est une concentration en mol/L.

Une concentration exprimée en mM est une concentration en mmol/L.

Dans un mode de réalisation de la composition, la concentration en protéine comportant au moins un fragment d'anticorps dans la formulation finale est supérieure ou égale à 50 mg/mL.

Dans un mode de réalisation de la composition, la concentration en protéine comportant au moins un fragment d'anticorps dans la formulation finale est comprise entre 50 et 350 mg/mL.

Dans un mode de réalisation de la composition, la concentration en protéine comportant au moins un fragment d'anticorps dans la formulation finale est supérieure ou égale à 80 mg/mL.

Dans un mode de réalisation de la composition, la concentration en protéine comportant au moins un fragment d'anticorps dans la formulation finale est comprise entre 80 et 350 mgjmL.

Dans un mode de réalisation de la composition, la concentration en protéine comportant au moins un fragment d'anticorps dans la formulation finale est comprise entre 80 et 300 mg/mL.

Dans un mode de réalisation de la composition, la concentration en protéine comportant au moins un fragment d'anticorps dans la formulation finale est supérieure ou égale à 100 mg/mL.

Dans un mode de réalisation de la composition, la concentration en protéine comportant au moins un fragment d'anticorps dans la formulation finale est comprise entre 100 et 350 mg/mL.

Dans un mode de réalisation de la composition, la concentration en protéine comportant au moins un fragment d'anticorps dans la formulation finale est comprise entre 100 et 300 mg/mL.

Dans un mode de réalisation de la composition, la concentration en protéine comportant au moins un fragment d'anticorps dans la formulation finale est supérieure ou égale à 150 mg/mL.

Dans un mode de réalisation de la composition, la concentration en protéine comportant au moins un fragment d'anticorps dans la formulation finale est comprise entre 150 et 300 mg/mL.

Dans un mode de réalisation de la composition, la concentration en protéine comportant au moins un fragment d'anticorps dans la formulation finale est comprise entre 150 et 250 mg/mL.

Dans un mode de réalisation de la composition, la concentration en protéine comportant au moins un fragment d'anticorps dans la formulation finale est comprise entre 80 et 250 mg/mL.

Dans un mode de réalisation de la composition, la concentration en protéine comportant au moins un fragment d'anticorps dans la formulation finale est comprise entre 100 et 250 mg/mL.

Dans un mode de réalisation de la composition, la concentration en protéine comportant au moins un fragment d'anticorps dans la formulation finale est comprise entre 80 et 220 mg/mL.

Dans un mode de réalisation de la composition, la concentration en protéine comportant au moins un fragment d'anticorps dans la formulation finale est comprise entre 105 et 220 mg/mL.

Dans un mode de réalisation de la composition, la concentration en protéine comportant au moins un fragment d'anticorps dans la formulation finale est comprise entre 125 et 220 mg/mL.

Dans un mode de réalisation de la composition, la concentration en protéine comportant au moins un fragment d'anticorps dans la formulation finale est comprise entre 140 et 220 mg/mL.

Dans un mode de réalisation de la composition, la concentration en protéine comportant au moins un fragment d'anticorps dans la formulation finale est comprise entre 150 et 220 mg/mL.

Dans un mode de réalisation de la composition, la concentration en agent réducteur de viscosité dans la formulation finale est supérieure ou égale à 10 mM.

Dans un mode de réalisation de la composition, la concentration en agent réducteur de viscosité dans la formulation finale est comprise entre 10 et 350 mM.

Dans un mode de réalisation de la composition, la concentration finale en composé réducteur de viscosité dans la formulation finale est comprise entre 50 et 350 mM.

Dans un mode de réalisation de la composition, la concentration finale en composé réducteur de viscosité dans la formulation finale est comprise entre 100 et 350 mM.

Dans un mode de réalisation de la composition, la concentration finale en composé réducteur de viscosité dans la formulation finale est comprise entre 50 et 300 mM.

Dans un mode de réalisation de la composition, la concentration finale en composé réducteur de viscosité dans la formulation finale est comprise entre 100 et 300 mM.

Dans un mode de réalisation de la composition, la concentration finale en composé réducteur de viscosité dans la formulation finale est comprise entre 50 et 250 mM.

Dans un mode de réalisation de la composition, la concentration finale en composé réducteur de viscosité dans la formulation finale est comprise entre 100 et 250 mM.

Dans un mode de réalisation de la composition, la concentration finale en composé réducteur de viscosité dans la formulation finale est comprise entre 50 et 200 mM.

Dans un mode de réalisation de la composition, la concentration finale en composé réducteur de viscosité dans la formulation finale est comprise entre 100 et 200 mM.

Dans un mode de réalisation de la composition, la protéine comportant au moins un fragment d'anticorps est choisie parmi les anticorps monoclonaux (mAbs), les anticorps polyclonaux, les protéines de fusion, les nanobodies, les anticorps bispécifiques et les anticorps couplés à des principes actifs cytotoxiques (ADC).

Dans un mode de réalisation de la composition, la protéine comportant au moins un fragment d'anticorps est un anticorps monoclonal.

Dans un mode de réalisation de la composition, l'anticorps monoclonal est un IgG.

Dans un mode de réalisation de la composition, l'anticorps monoclonal est un IgA.

Dans un mode de réalisation de la composition, l'anticorps monoclonal est un IgM.

Dans un mode de réalisation de la composition, l'anticorps monoclonal est un IgE.

Dans un mode de réalisation de la composition, l'anticorps monoclonal est un IgD.

Dans un mode de réalisation de la composition, l'anticorps monoclonal est un IgG1.

Dans un mode de réalisation de la composition, l'anticorps monoclonal est un IgG2.

Dans un mode de réalisation de la composition, l'anticorps monoclonal est un IgG3.

Dans un mode de réalisation de la composition, l'anticorps monoclonal est un IgG4.

Dans un mode de réalisation de la composition, l'anticorps monoclonal est un IgA1.

Dans un mode de réalisation de la composition, l'anticorps monoclonal est un IgA2.

Dans un mode de réalisation de la composition, la protéine comportant au moins un fragment d'anticorps est un anticorps polyclonal.

Dans un mode de réalisation de la composition, la protéine comportant au moins un fragment d'anticorps est une protéine de fusion.

Dans un mode de réalisation de la composition, la protéine comportant au moins un fragment d'anticorps est un nanobody.

Dans un mode de réalisation de la composition, la protéine comportant au moins un fragment d'anticorps est un anticorps bispécifique.

Dans un mode de réalisation de la composition, la protéine comportant au moins un fragment d'anticorps est un anticorps couplé à un principe actif cytotoxique.

Dans un mode de réalisation de la composition, la composition comprend en outre un acide pharmaceutiquement acceptable.

Dans un mode de réalisation de la composition, l'acide est choisi dans le groupe constitué par : l'acide hydrochlorique, l'acide phosphorique, l'acide citrique, l'acide acétique, l'acide ascorbique, l'acide éthylène diamine tétraacétique (appelé aussi EDTA), l'acide tartrique.

Dans un mode de réalisation de la composition, la composition comprend en outre une base pharmaceutiquement acceptable.

Dans un mode de réalisation de la composition, la base est choisie dans le groupe constitué des bases inorganiques formées à partir des métaux tels que le sodium, le potassium, le calcium, le magnésium.

Dans un mode de réalisation de la composition, la base inorganique est choisie dans le groupe constitué par la soude (NaOH), la potasse (KOH), l'hydroxyde de magnésium (Mg(OH)₂).

Additionnellement, les acides et/ou bases pharmaceutiquement acceptables incluent ceux ou celles dérivés d'acides aminés comme par exemple l'histidine, l'arginine ou la glycine.

Dans un mode de réalisation de la composition, la composition comprend en outre un sel inorganique pharmaceutiquement acceptable.

Dans un mode de réalisation de la composition, le sel est choisi dans le groupe constitué par : le sel de sodium, le sel de potassium, le chlorure d'étain(II).

Dans un mode de réalisation de la composition, la composition comprend en outre un tampon pharmaceutiquement acceptable. Ces tampons pharmaceutiquement acceptables incluent ceux qui sont dérivés des sels des acides et des bases précédemment citées ou de la combinaison de ces derniers.

Dans un mode de réalisation de la composition, le tampon est choisi dans le groupe constitué par : phosphate de sodium monobasique (appelé aussi phosphate monosodique)/phosphate de sodium dibasique (appelé aussi phosphate disodique), phosphate de potassium monobasique (appelé aussi phosphate monopotassique)/phosphate de sodium dibasique (appelé aussi phosphate disodique)/sel de sodium, acide acétique/acétate de sodium, acide citrique/citrate de sodium, hydrochlorate de L-Histidine/Histidine, hydrochlorate de Glycine/Glycine.

Dans un mode de réalisation de la composition, la composition comprend en outre un diluant pharmaceutique.

Dans un mode de réalisation de la composition, le diluant est choisi dans le groupe constitué par : une eau stérile pour injection ou une eau bactériostatique pour injection, une solution tamponnée en pH (telle qu'un tampon phosphate salin par exemple), une solution saline pour injection (NaCl 0,9%). Alternativement le diluent peut être une solution aqueuse saline et/ou un tampon.

Dans un mode de réalisation de la composition, la composition comprend en outre un conservateur pharmaceutique.

Dans un mode de réalisation de la composition, le conservateur est choisi dans le groupe constitué par l'alcool benzylique, le phénol, le m-crésol ou la povidone.

Dans un mode de réalisation de la composition, la composition comprend en outre un tensio-actif.

Dans un mode de réalisation de la composition, le tensio-actif est choisi dans le groupe constitué par : le polysorbate 20 (appelé aussi PS20 ou Tween20), le polysorbate 80 (appelé aussi PS80 Tween80), le Pluronic F-68, les « Brij » ainsi que les alkylglucosides tels que le n-dodecyl-β-D-maltoglucoside (DDM).

Dans un mode de réalisation, la composition comprend en outre un lyoprotecteur et/ou un sucre pharmaceutiquement acceptable.

Dans un mode de réalisation de la composition, le lyoprotecteur ou le sucre pharmaceutiquement acceptable est choisi dans le groupe constitué par : l'α-tréhalose, le saccharose (appelé aussi sucrose), le maltose, le mannitol, le sorbitol, le dextran. Alternativement, les lyoprotecteurs incluent des acides aminés tels que l'histidine.

Tout particulièrement, la composition est une solution aqueuse comprenant,
- au moins au moins une protéine comprenant au moins un fragment d'anticorps,
- au moins un tampon,
- au moins un tensioactif, et
- au moins un diluant.

Dans un mode de réalisation, le composition comprend en outre au moins un lyoprotecteur ou sucre pharmaceutiquement acceptable.

Dans un mode de réalisation, le composition comprend en outre un acide ou une base.

Ledit acide ou ladite base est en particulier destiné à ajuster le pH de la solution, en particulier ledit acide est le HCl et ladite base est le NaOH.

Les composés compris dans la composition sont pharamaceutiquement acceptables.

Dans un mode de réalisation, l'invention concerne une composition pour injection comprenant au moins une protéine comprenant au moins un fragment d'anticorps, du phosphate de sodium (monobasique, monohydrate), du phosphate de sodium (dibasique, dihydrate), du sucrose, un polysorbate et au moins un agent réducteur de viscosité, en solution dans de l'eau pour injection.

Dans un mode de réalisation, l'invention concerne une composition pour injection comprenant au moins une protéine comprenant au moins un fragment d'anticorps, du phosphate de sodium (monobasique, anhydre), du phosphate de sodium (dibasique, anhydre), du dihydrate d' α,α-trehalose, un polysorbate et au moins un agent réducteur de viscosité, en solution dans de l'eau pour injection.

L'invention concerne également un procédé d'abaissement de la viscosité d'une solution aqueuse difficilement injectable d'au moins une protéine comportant au moins un fragment d'anticorps dont la concentration est supérieure ou égale à 50 mg/mL et dont le pH est compris entre 5 et 8, d'une valeur d'au moins 10%, consistant à préparer une solution aqueuse comprenant :
- ladite protéine comportant au moins un fragment d'anticorps à une concentration supérieure ou égale à 50 mg/mL, et au moins
- un agent réducteur de viscosité soluble dans l'eau, choisi dans le groupe constitué par la cytidine, la 2'-deoxycytidine, l'uridine, la 2'-deoxyuridine, la thymidine et la ribothymidine , seule ou en mélange.

Dans un mode de réalisation du procédé, le composé réducteur de viscosité est choisi dans le groupe constitué par la cytidine, l'uridine et la ribothymidine, seule ou en mélange.

Dans un mode de réalisation du procédé, le composé réducteur de viscosité est choisi dans le groupe constitué par la 2'-deoxycytidine, la 2'-deoxyuridine et la thymidine, seule ou en mélange.

Dans un mode de réalisation du procédé, le composé réducteur de viscosité est choisi dans le groupe constitué par la cytidine, et la 2'-deoxycytidine, seule ou en mélange.

Dans un mode de réalisation du procédé, le composé réducteur de viscosité est choisi dans le groupe constitué par l'uridine, et la 2'-deoxyuridine seule ou en mélange.

Dans un mode de réalisation du procédé, le composé réducteur de viscosité est choisi dans le groupe constitué par la thymidine et la ribothymidine seule ou en mélange.

Dans un mode de réalisation du procédé, le composé réducteur de viscosité est choisi dans le groupe constitué de la cytidine, la 2'-deoxycytidine et l'uridine, seule ou en mélange.

Dans un mode de réalisation du procédé, le composé réducteur de viscosité est choisi dans le groupe constitué par la 2'-deoxyuridine, la thymidine et la ribothymidine, seule ou en mélange.

Dans un mode de réalisation, le composé réducteur de viscosité est la cytidine.

Dans un mode de réalisation, le composé réducteur de viscosité est la 2'-deoxycytidine.

Dans un mode de réalisation, le composé réducteur de viscosité est l'uridine.

Dans un mode de réalisation, le composé réducteur de viscosité est la 2'-deoxyuridine.

Dans un mode de réalisation, le composé réducteur de viscosité est la thymidine.

Dans un mode de réalisation, le composé réducteur de viscosité est la ribothymidine.

Dans un mode de réalisation du procédé, la viscosité est abaissée d'une valeur d'au moins 15 %.

Dans un mode de réalisation du procédé, la viscosité est abaissée d'une valeur comprise entre 10 et 90%.

Dans un mode de réalisation du procédé, la viscosité est abaissée d'une valeur comprise entre 20 et 90%.

Dans un mode de réalisation du procédé, la viscosité est abaissée d'une valeur comprise entre 30 et 85%.

Dans un mode de réalisation du procédé, la viscosité est abaissée d'une valeur comprise entre 35 et 80%.

Dans un mode de réalisation du procédé, la viscosité est abaissée d'une valeur comprise entre 35 et 77%.

Dans un mode de réalisation du procédé, la viscosité est abaissée d'une valeur comprise entre 60 et 90%.

Dans un mode de réalisation du procédé, la viscosité est abaissée d'une valeur comprise entre 60 et 80%.

Dans un mode de réalisation du procédé, la concentration en protéine comportant au moins un fragment d'anticorps dans la formulation finale est comprise entre 50 et 350 mg/mL.

Dans un mode de réalisation du procédé, la concentration en protéine comportant au moins un fragment d'anticorps dans la formulation finale est supérieure ou égale à 80 mg/mL.

Dans un mode de réalisation du procédé, la concentration en protéine comportant au moins un fragment d'anticorps dans la formulation finale est comprise entre 80 et 350 mg/mL.

Dans un mode de réalisation du procédé, la concentration en protéine comportant au moins un fragment d'anticorps dans la formulation finale est supérieure ou égale à 100 mg/mL.

Dans un mode de réalisation du procédé, la concentration en protéine comportant au moins un fragment d'anticorps dans la formulation finale est comprise entre 100 et 350 mg/mL.

Dans un mode de réalisation du procédé, la concentration en protéine comportant au moins un fragment d'anticorps dans la formulation finale est comprise entre 80 et 300 mg/mL.

Dans un mode de réalisation du procédé, la concentration en protéine comportant au moins un fragment d'anticorps dans la formulation finale est comprise entre 100 et 300 mg/mL.

Dans un mode de réalisation du procédé, la concentration en protéine comportant au moins un fragment d'anticorps dans la formulation finale est supérieure ou égale à 150 mg/mL.

Dans un mode de réalisation du procédé, la concentration en protéine comportant au moins un fragment d'anticorps dans la formulation finale est comprise entre 150 et 300 mg/mL.

Dans un mode de réalisation du procédé, la concentration en protéine comportant au moins un fragment d'anticorps dans la formulation finale est comprise entre 150 et 250 mg/mL.

Dans un mode de réalisation du procédé, la concentration en protéine comportant au moins un fragment d'anticorps dans la formulation finale est comprise entre 80 et 250 mg/mL.

Dans un mode de réalisation du procédé, la concentration en protéine comportant au moins un fragment d'anticorps dans la formulation finale est comprise entre 100 et 250 mg/mL.

Dans un mode de réalisation du procédé, la concentration en protéine comportant au moins un fragment d'anticorps dans la formulation finale est comprise entre 80 et 220 mg/mL.

Dans un mode de réalisation du procédé, la concentration en protéine comportant au moins un fragment d'anticorps dans la formulation finale est comprise entre 105 et 220 mg/mL.

Dans un mode de réalisation du procédé, la concentration en protéine comportant au moins un fragment d'anticorps dans la formulation finale est comprise entre 125 et 220 mg/mL.

Dans un mode de réalisation du procédé, la concentration en protéine comportant au moins un fragment d'anticorps dans la formulation finale est comprise entre 140 et 220 mg/mL.

Dans un mode de réalisation du procédé, la concentration en protéine comportant au moins un fragment d'anticorps dans la formulation finale est comprise entre 150 et 220 mg/mL.

Dans un mode de réalisation du procédé, ladite protéine comportant au moins un fragment d'anticorps est présente dans la formulation finale à une concentration supérieure ou égale à 50 mg/mL, et la viscosité est abaissée d'une valeur d'au moins 15% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration comprise entre 50 et 350 mg/mL, et la viscosité est abaissée d'une valeur d'au moins 15% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration supérieure ou égale à 100 mg/mL, et la viscosité est abaissée d'une valeur d'au moins 25% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration comprise entre 100 et 350 mg/mL, et la viscosité est abaissée d'une valeur d'au moins 25% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration supérieure ou égale à 150 mg/mL, et la viscosité est abaissée d'une valeur d'au moins 35% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration comprise entre 150 et 350 mg/mL, et la viscosité est abaissée d'une valeur d'au moins 35% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration supérieure ou égale à 200 mg/mL, et la viscosité est abaissée d'une valeur d'au moins 40% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, ladite protéine comportant au moins un fragment d'anticorps est présente dans la formulation finale à une concentration comprise entre 200 et 350 mg/mL, et la viscosité est abaissée d'une valeur d'au moins 40% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation du procédé, la viscosité d'une solution aqueuse difficilement injectable d'au moins une protéine comportant au moins un fragment d'anticorps d'au moins 15 cP, est abaissé d'une valeur d'au moins 10% par ledit agent réducteur de viscosité.

Dans un mode de réalisation du procédé, la viscosité d'une solution aqueuse difficilement injectable d'au moins une protéine comportant au moins un fragment d'anticorps d'au moins 50 cP, est abaissé d'une valeur d'au moins 15% par ledit agent réducteur de viscosité.

Dans un mode de réalisation du procédé, la viscosité d'une solution aqueuse difficilement injectable d'au moins une protéine comportant au moins un fragment d'anticorps d'au moins 100 cP, est abaissé d'une valeur d'au moins 20% par l'agent réducteur de viscosité.

Dans un mode de réalisation du procédé, la viscosité d'une solution aqueuse difficilement injectable d'au moins une protéine comportant au moins un fragment d'anticorps d'au moins 150 cP, est abaissé d'une valeur d'au moins 25% par ledit agent réducteur de viscosité.

Dans un mode de réalisation du procédé, la viscosité d'une solution aqueuse difficilement injectable d'au moins une protéine comportant au moins un fragment d'anticorps d'au moins 200 cP, est abaissé d'une valeur d'au moins 35% par ledit agent réducteur de viscosité.

Dans un mode de réalisation du procédé, la concentration en agent réducteur de viscosité dans la formulation finale est supérieure ou égale à 10 mM.

Dans un mode de réalisation du procédé, la concentration en agent réducteur de viscosité dans la formulation finale est comprise entre 10 et 350 mM.

Dans un mode de réalisation du procédé, la concentration finale en composé réducteur de viscosité dans la formulation finale est comprise entre 50 et 350 mM.

Dans un mode de réalisation du procédé, la concentration finale en composé réducteur de viscosité dans la formulation finale est comprise entre 100 et 350 mM.

Dans un mode de réalisation du procédé, la concentration finale en composé réducteur de viscosité dans la formulation finale est comprise entre 50 et 300 mM.

Dans un mode de réalisation du procédé, la concentration finale en composé réducteur de viscosité dans la formulation finale est comprise entre 100 et 300 mM.

Dans un mode de réalisation du procédé, la concentration finale en composé réducteur de viscosité dans la formulation finale est comprise entre 50 et 250 mM.

Dans un mode de réalisation du procédé, la concentration finale en composé réducteur de viscosité dans la formulation finale est comprise entre 100 et 250 mM.

Dans un mode de réalisation du procédé, la concentration finale en composé réducteur de viscosité dans la formulation finale est comprise entre 50 et 200 mM.

Dans un mode de réalisation du procédé, la concentration finale en composé réducteur de viscosité dans la formulation finale est comprise entre 100 et 200 mM.

Dans un mode de réalisation du procédé, le pH de la solution aqueuse difficilement injectable d'au moins une protéine comportant au moins un fragment d'anticorps est compris entre 5 et 8.

Dans un mode de réalisation du procédé, le pH de la solution aqueuse difficilement injectable d'au moins une protéine comportant au moins un fragment d'anticorps est compris entre 5 et 6,5.

Dans un mode de réalisation du procédé, le pH de la solution aqueuse difficilement injectable d'au moins une protéine comportant au moins un fragment d'anticorps est compris entre 5,5 et 6,5.

Dans un mode de réalisation du procédé, le pH de la solution aqueuse difficilement injectable d'au moins une protéine comportant au moins un fragment d'anticorps est compris entre 6 et 8.

Dans un mode de réalisation du procédé, le pH de la solution aqueuse difficilement injectable d'au moins une protéine comportant au moins un fragment d'anticorps est compris entre 6 et 7,5.

Dans un mode de réalisation du procédé, le pH de la solution aqueuse difficilement injectable d'au moins une protéine comportant au moins un fragment d'anticorps est compris entre 6 et 7.

Dans un mode de réalisation du procédé, la protéine comportant au moins un fragment d'anticorps est choisie parmi les anticorps monoclonaux (mAbs), les anticorps polyclonaux, les protéines de fusion, les nanobodies, les anticorps bispécifiques et les anticorps couplés à des principes actifs cytotoxiques (ADC).

Dans un mode de réalisation, la protéine comportant au moins un fragment d'anticorps est un anticorps monoclonal.

Dans un mode de réalisation du procédé, l'anticorps monoclonal est un IgG.

Dans un mode de réalisation du procédé, l'anticorps monoclonal est un IgA.

Dans un mode de réalisation du procédé, l'anticorps monoclonal est un IgM.

Dans un mode de réalisation du procédé, l'anticorps monoclonal est un IgE.

Dans un mode de réalisation du procédé, l'anticorps monoclonal est un IgD.

Dans un mode de réalisation du procédé, l'anticorps monoclonal est un IgG1.

Dans un mode de réalisation du procédé, l'anticorps monoclonal est un IgG2.

Dans un mode de réalisation du procédé, l'anticorps monoclonal est un IgG3.

Dans un mode de réalisation du procédé, l'anticorps monoclonal est un IgG4.

Dans un mode de réalisation du procédé, l'anticorps monoclonal est un IgA1.

Dans un mode de réalisation du procédé, l'anticorps monoclonal est un IgA2.

Dans un mode de réalisation du procédé, la protéine comportant au moins un fragment d'anticorps est un anticorps polyclonal.

Dans un mode de réalisation du procédé, la protéine comportant au moins un fragment d'anticorps est une protéine de fusion.

Dans un mode de réalisation du procédé, la protéine comportant au moins un fragment d'anticorps est un nanobody.

Dans un mode de réalisation du procédé, la protéine comportant au moins un fragment d'anticorps est un anticorps bispécifique.

Dans un mode de réalisation du procédé, la protéine comportant au moins un fragment d'anticorps est un anticorps couplé un principe actif cytotoxique.

Dans un mode de réalisation du procédé, le procédé consiste en le mélange d'une solution aqueuse contenant le ou les agents réducteurs de viscosité avec une solution aqueuse contenant la protéine comportant au moins un fragment d'anticorps.

Dans un mode de réalisation du procédé, le procédé consiste en la solubilisation d'un lyophilisat contenant la protéine comportant au moins un fragment d'anticorps et le ou les agents réducteurs de viscosité à l'aide d'une solution aqueuse.

Dans un mode de réalisation du procédé, le procédé consiste en la solubilisation d'un lyophilisat contenant la protéine comportant au moins un fragment d'anticorps et/ou le ou les agents réducteurs de viscosité à l'aide d'une solution aqueuse contenant la protéine comportant au moins un fragment d'anticorps et/ou le ou les agents réducteurs de viscosité.

Dans un mode de réalisation du procédé, le procédé consiste en la préparation d'un mélange à partir d'une solution aqueuse contenant la protéine comportant au moins un fragment d'anticorps et d'une solution aqueuse contenant le ou les agents réducteurs de viscosité, suivie de la concentration de ce mélange, par diafiltration/ultrafiltration, ou encore par lyophilisation et reprise du lyophilisat à l'aide d'une solution aqueuse contenant éventuellement le ou les agents réducteurs de viscosité et dont le volume est inférieur à celui du mélange d'origine, pour obtenir une solution dont la concentration en protéine est supérieure à la concentration en protéine de la solution de protéine de départ.

L'invention concerne également l'utilisation d'au moins un agent réducteur de viscosité soluble dans l'eau, choisi dans le groupe constitué par la cytidine, la 2'-deoxycytidine, l'uridine, la 2'-deoxyuridine, la thymidine et la ribothymidine, seule ou en mélange à une concentration dans la formulation finale supérieure ou égale à 10 mM, pour abaisser la viscosité d'une solution aqueuse difficilement injectable d'au moins une protéine comportant au moins un fragment d'anticorps dont la concentration est supérieure ou égale à 50 mg/mL, et dont le pH est compris entre 5 et 8, d'une valeur d'au moins 10% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, le composé réducteur de viscosité est choisi dans le groupe constitué par la cytidine, l'uridine et la ribothymidine, seule ou en mélange.

Dans un mode de réalisation de l'utilisation, le composé réducteur de viscosité est choisi dans le groupe constitué par la 2'-deoxycytidine, la 2'-deoxyuridine et la thymidine, seule ou en mélange.

Dans un mode de réalisation de l'utilisation, le composé réducteur de viscosité est choisi dans le groupe constitué par la cytidine et la 2'-deoxycytidine, seule ou en mélange.

Dans un mode de réalisation de l'utilisation, le composé réducteur de viscosité est choisi dans le groupe constitué par l'uridine et la 2'-deoxyuridine, seule ou en mélange.

Dans un mode de réalisation de l'utilisation, le composé réducteur de viscosité est choisi dans le groupe constitué par la thymidine et la ribothymidine, seule ou en mélange.

Dans un mode de réalisation de l'utilisation, le composé réducteur de viscosité est choisi dans le groupe constitué de la cytidine, la 2'-deoxycytidine et l'uridine, seule ou en mélange.

Dans un mode de réalisation de l'utilisation, le composé réducteur de viscosité est choisi dans le groupe constitué par la 2'-deoxyuridine, la thymidine et la ribothymidine, seule ou en mélange.

Dans un mode de réalisation de l'utilisation, le composé réducteur de viscosité est la cytidine.

Dans un mode de réalisation de l'utilisation, le composé réducteur de viscosité est la 2'-deoxycytidine.

Dans un mode de réalisation de l'utilisation, le composé réducteur de viscosité est l'uridine.

Dans un mode de réalisation de l'utilisation, le composé réducteur de viscosité est la 2'-deoxyuridine.

Dans un mode de réalisation de l'utilisation, le composé réducteur de viscosité est la thymidine.

Dans un mode de réalisation de l'utilisation, le composé réducteur de viscosité est la ribothymidine.

Dans un mode de réalisation de l'utilisation, la viscosité est abaissée d'une valeur d'au moins 15 %.

Dans un mode de réalisation de l'utilisation, la viscosité est abaissée d'une valeur comprise entre 10 et 90%.

Dans un mode de réalisation de l'utilisation, la viscosité est abaissée d'une valeur comprise entre 20 et 90%.

Dans un mode de réalisation de l'utilisation, la viscosité est abaissée d'une valeur comprise entre 30 et 85%.

Dans un mode de réalisation de l'utilisation, la viscosité est abaissée d'une valeur comprise entre 35 et 80%.

Dans un mode de réalisation de l'utilisation, la viscosité est abaissée d'une valeur comprise entre 35 et 77%.

Dans un mode de réalisation de l'utilisation, la viscosité est abaissée d'une valeur comprise entre 60 et 90%.

Dans un mode de réalisation de l'utilisation, la viscosité est abaissée d'une valeur comprise entre 60 et 80%.

Dans un mode de réalisation de l'utilisation, la concentration en protéine comportant au moins un fragment d'anticorps dans la formulation finale est comprise entre 50 et 350 mg/mL.

Dans un mode de réalisation de l'utilisation, la concentration en protéine comportant au moins un fragment d'anticorps dans la formulation finale est supérieure ou égale à 80 mg/mL.

Dans un mode de réalisation de l'utilisation, la concentration en protéine comportant au moins un fragment d'anticorps dans la formulation finale est comprise entre 80 et 350 mg/mL.

Dans un mode de réalisation de l'utilisation, la concentration en protéine comportant au moins un fragment d'anticorps dans la formulation finale est supérieure ou égale à 100 mg/mL.

Dans un mode de réalisation de l'utilisation, la concentration en protéine comportant au moins un fragment d'anticorps dans la formulation finale est comprise entre 100 et 350 mg/mL.

Dans un mode de réalisation de l'utilisation, la concentration en protéine comportant au moins un fragment d'anticorps dans la formulation finale est comprise entre 80 et 300 mg/mL.

Dans un mode de réalisation de l'utilisation, la concentration en protéine comportant au moins un fragment d'anticorps dans la formulation finale est comprise entre 100 et 300 mg/mL.

Dans un mode de réalisation de l'utilisation, la concentration en protéine comportant au moins un fragment d'anticorps dans la formulation finale est supérieure ou égale à 150 mg/mL.

Dans un mode de réalisation de l'utilisation, la concentration en protéine comportant au moins un fragment d'anticorps dans la formulation finale est comprise entre 150 et 300 mg/mL.

Dans un mode de réalisation de l'utilisation, la concentration en protéine comportant au moins un fragment d'anticorps dans la formulation finale est comprise entre 150 et 250 mg/mL.

Dans un mode de réalisation de l'utilisation, la concentration en protéine comportant au moins un fragment d'anticorps dans la formulation finale est comprise entre 80 et 250 mg/mL.

Dans un mode de réalisation de l'utilisation, la concentration en protéine comportant au moins un fragment d'anticorps dans la formulation finale est comprise entre 100 et 250 mg/mL.

Dans un mode de réalisation de l'utilisation, la concentration en protéine comportant au moins un fragment d'anticorps dans la formulation finale est comprise entre 80 et 220 mg/mL.

Dans un mode de réalisation de l'utilisation, la concentration en protéine comportant au moins un fragment d'anticorps dans la formulation finale est comprise entre 105 et 220 mg/mL.

Dans un mode de réalisation de l'utilisation, la concentration en protéine comportant au moins un fragment d'anticorps dans la formulation finale est comprise entre 125 et 220 mg/mL.

Dans un mode de réalisation de l'utilisation, la concentration en protéine comportant au moins un fragment d'anticorps dans la formulation finale est comprise entre 140 et 220 mg/mL.

Dans un mode de réalisation de l'utilisation, la concentration en protéine comportant au moins un fragment d'anticorps dans la formulation finale est comprise entre 150 et 220 mg/mL.

Dans un mode de réalisation de l'utilisation, ladite protéine comportant au moins un fragment d'anticorps est présente dans la formulation finale à une concentration supérieure ou égale à 50 mg/mL, et la viscosité est abaissée d'une valeur d'au moins 15% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration comprise entre 50 et 350 mg/mL, et la viscosité est abaissée d'une valeur d'au moins 15% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration supérieure ou égale à 100 mg/mL, et la viscosité est abaissée d'une valeur d'au moins 25% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration comprise entre 100 et 350 mg/mL, et la viscosité est abaissée d'une valeur d'au moins 25% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration supérieure ou égale à 150 mg/mL, et la viscosité est abaissée d'une valeur d'au moins 35% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration comprise entre 150 et 350 mg/mL, et la viscosité est abaissée d'une valeur d'au moins 35% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration supérieure ou égale à 200 mg/mL, et le la viscosité est abaissée d'une valeur d'au moins 40% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, ladite protéine comportant au moins un fragment d'anticorps est présente dans la formulation finale à une concentration comprise entre 200 et 350 mg/mL, et le la viscosité est abaissée d'une valeur d'au moins 40% par rapport à la viscosité d'une solution aqueuse difficilement injectable d'au moins une protéine comportant au moins un fragment d'anticorps de même concentration et de même pH ne contenant pas ledit agent réducteur de viscosité.

Dans un mode de réalisation de l'utilisation, la viscosité d'une solution aqueuse difficilement injectable d'au moins une protéine comportant au moins un fragment d'anticorps d'au moins 15 cP, est abaissé d'une valeur d'au moins 10% par ledit agent réducteur de viscosité.

Dans un mode de réalisation de l'utilisation, la viscosité d'une solution aqueuse difficilement injectable d'au moins une protéine comportant au moins un fragment d'anticorps d'au moins 50 cP, est abaissé d'une valeur d'au moins 15% par ledit agent réducteur de viscosité.

Dans un mode de réalisation de l'utilisation, la viscosité d'une solution aqueuse difficilement injectable d'au moins une protéine comportant au moins un fragment d'anticorps d'au moins 100 cP, est abaissé d'une valeur d'au moins 20% par l'agent réducteur de viscosité.

Dans un mode de réalisation de l'utilisation, la viscosité d'une solution aqueuse difficilement injectable d'au moins une protéine comportant au moins un fragment d'anticorps d'au moins 150 cP, est abaissé d'une valeur d'au moins 25% par ledit agent réducteur de viscosité.

Dans un mode de réalisation de l'utilisation, la viscosité d'une solution aqueuse difficilement injectable d'au moins une protéine comportant au moins un fragment d'anticorps d'au moins 200 cP, est abaissé d'une valeur d'au moins 35% par ledit agent réducteur de viscosité.

Dans un mode de réalisation de l'utilisation, la concentration finale en composé réducteur de viscosité dans la formulation finale est comprise entre 10 et 350 mM.

Dans un mode de réalisation de l'utilisation, la concentration finale en composé réducteur de viscosité dans la formulation finale est comprise entre 50 et 350 mM.

Dans un mode de réalisation de l'utilisation, la concentration finale en composé réducteur de viscosité dans la formulation finale est comprise entre 100 et 350 mM.

Dans un mode de réalisation de l'utilisation, la concentration finale en composé réducteur de viscosité dans la formulation finale est comprise entre 50 et 300 mM.

Dans un mode de réalisation de l'utilisation, la concentration finale en composé réducteur de viscosité dans la formulation finale est comprise entre 100 et 300 mM.

Dans un mode de réalisation de l'utilisation, la concentration finale en composé réducteur de viscosité dans la formulation finale est comprise entre 50 et 250 mM.

Dans un mode de réalisation de l'utilisation, la concentration finale en composé réducteur de viscosité dans la formulation finale est comprise entre 100 et 250 mM.

Dans un mode de réalisation de l'utilisation, la concentration finale en composé réducteur de viscosité dans la formulation finale est comprise entre 50 et 200 mM.

Dans un mode de réalisation de l'utilisation, la concentration finale en composé réducteur de viscosité dans la formulation finale est comprise entre 100 et 200 mM.

Dans un mode de réalisation de l'utilisation, le pH de la solution aqueuse difficilement injectable d'au moins une protéine comportant au moins un fragment d'anticorps est compris entre 5 et 6,5.

Dans un mode de réalisation de l'utilisation, le pH de la solution aqueuse difficilement injectable d'au moins une protéine comportant au moins un fragment d'anticorps est compris entre 5,5 et 6,5.

Dans un mode de réalisation de l'utilisation, le pH de la solution aqueuse difficilement injectable d'au moins une protéine comportant au moins un fragment d'anticorps est compris entre 6 et 8.

Dans un mode de réalisation de l'utilisation, le pH de la solution aqueuse difficilement injectable d'au moins une protéine comportant au moins un fragment d'anticorps est compris entre 6 et 7,5.

Dans un mode de réalisation de l'utilisation, le pH de la solution aqueuse difficilement injectable d'au moins une protéine comportant au moins un fragment d'anticorps est compris entre 6 et 7.

Dans un mode de réalisation de l'utilisation, la protéine comportant au moins un fragment d'anticorps est choisie parmi les anticorps monoclonaux (mAbs), les anticorps polyclonaux, les protéines de fusion, les nanobodies, les anticorps bispécifiques et les anticorps couplés des principes actifs cytotoxiques (ADC).

Dans un mode de réalisation de l'utilisation, la protéine comportant au moins un fragment d'anticorps est un anticorps monoclonal.

Dans un mode de réalisation de l'utilisation, l'anticorps monoclonal est un IgG.

Dans un mode de réalisation de l'utilisation, l'anticorps monoclonal est un IgA.

Dans un mode de réalisation de l'utilisation, l'anticorps monoclonal est un IgM.

Dans un mode de réalisation de l'utilisation, l'anticorps monoclonal est un IgE.

Dans un mode de réalisation de l'utilisation, l'anticorps monoclonal est un IgD.

Dans un mode de réalisation de l'utilisation, l'anticorps monoclonal est un IgG1.

Dans un mode de réalisation de l'utilisation, l'anticorps monoclonal est un IgG2.

Dans un mode de réalisation de l'utilisation, l'anticorps monoclonal est un IgG3.

Dans un mode de réalisation de l'utilisation, l'anticorps monoclonal est un IgG4.

Dans un mode de réalisation de l'utilisation, l'anticorps monoclonal est un IgA1.

Dans un mode de réalisation de l'utilisation, l'anticorps monoclonal est un IgA2.

Dans un mode de réalisation de l'utilisation, la protéine comportant au moins un fragment d'anticorps est un anticorps polyclonal.

Dans un mode de réalisation de l'utilisation, la protéine comportant au moins un fragment d'anticorps est une protéine de fusion.

Dans un mode de réalisation de l'utilisation, la protéine comportant au moins un fragment d'anticorps est un nanobody.

Dans un mode de réalisation de l'utilisation, la protéine comportant au moins un fragment d'anticorps est un anticorps bispécifique.

Dans un mode de réalisation de l'utilisation, la protéine comportant au moins un fragment d'anticorps est un anticorps couplé à un principe actif cytotoxique.

L'invention est illustrée par les exemples suivants. Ces exemples démontrent l'effet technique des composés réducteurs de viscosité utilisés selon l'invention et les comparent avec les composés de l'art antérieur.

### EXEMPLE 1 - Préparation de solution aqueuses mères.

**Tableau I**

| **Code** | **Nom** | **Structure** | **Fournisseur Référence** | **Numéro CAS** |
|---|---|---|---|---|
| Arg | Sel chlorhydrate de la L-arginine | 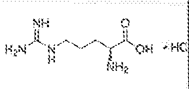 | Sigma 11039 | CAS# 1119-34-2 |
| Cyt | Cytidine | 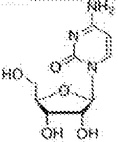 | Sigma C4654 | CAS# 65-46-3 |
| Deoxy cyt | 2'-Deoxycytidine | 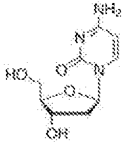 | SIGMA D3897 | CAS# 951-77-9 |
| Urid | Uridine | 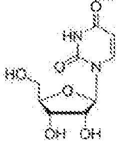 | SIGMA U3750 | CAS# 58-96-8 |
| Deoxy urid | 2'-Deoxyuridine | 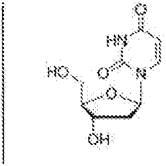 | SIGMA D5412 | CAS# 951-78-0 |
| Thym | Thymidine | 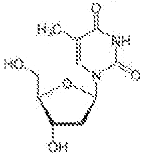 | SIGMA T9250 | CAS# 50-89-5 |
| Ribot hym | Ribothymidine | 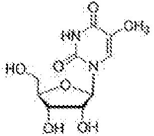 | SIGMA M8905 | CAS# 1463-10-1 |
| NaCl | Chlorure de sodium | Na⁺Cl⁻ | Cooper 171 0500 | CAS# 7647-14-5 |
| ATP | Sel de sodium de l'adénosine 5'-triphosphate | 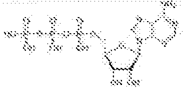 | SIGMA A3377 | CAS# 56-65-5 |

### Solution de sel chlorhydrate de la L-arginine

Le sel chlorhydrate de la L-arginine a été obtenu chez Sigma-Aldrich (Ref Sigma 11039, CAS# 1119-34-2) et a été solubilisé dans l'eau Milli-Q^{®} en ajustant le pH entre 5,5 et 8 de sorte à obtenir une solution à 994 mM en sel chlorhydrate de la L-arginine.

### Solution de cytidine

La cytidine a été obtenue chez Sigma-Aldrich (Ref C4654, CAS# 65-46-3) et a été solubilisée dans l'eau Milli-Q^{®} en ajustant le pH entre 5 et 8 de sorte à obtenir une solution à 905 mM en cytidine.

### Solution de 2'-deoxycytidine

La 2'-deoxycytidine a été obtenue chez Sigma-Aldrich (Ref Aldrich D3897, CAS# 951-77-9) et a été solubilisée dans l'eau Milli-Q^{®} en ajustant le pH entre 5 et 8 de sorte à obtenir une solution à 1120 mM en 2'-deoxycytidine.

### Solution d'uridine

L'uridine a été obtenue chez Sigma-Aldrich (Ref Aldrich U3750, CAS# 58-96-8) et a été solubilisée dans l'eau Milli-Q^{®} en ajustant le pH entre 5 et 8 de sorte à obtenir une solution à 1000 mM en uridine.

### Solution de 2'-deoxyuridine

La 2'-deoxyuridine a été obtenue chez SIGMA (Ref D5412, CAS# 951-78-0) et a été solubilisée dans l'eau Milli-Q^{®} en ajustant le pH entre 5 et 8 de sorte à obtenir une solution à 200 mM en 2'-deoxyuridine.

### Solution de thymidine

La thymidine a été obtenue chez SIGMA (Ref T9250, CAS# 50-89-5) et a été solubilisée dans l'eau Milli-Q® en ajustant le pH entre 5 et 8 de sorte à obtenir une solution à 20 mM en thymidine.

### Solution de ribothymidine

La ribothymidine a été obtenue chez SIGMA (Ref M8905, CAS# 1463-10-1) et a été solubilisée dans l'eau Milli-Q® en ajustant le pH entre 5 et 8 de sorte à obtenir une solution à 200 mM en ribothymidine.

### Solution de chlorure de sodium

Le chlorure de sodium a été obtenu chez Cooper (Ref Cooper 171 0500, CAS# 7647-14-5) dans l'eau Milli-Q^{®} de sorte à obtenir une solution à 2500 mM en sel de NaCl.

### Solution de sel de sodium de l'adénosine 5'-triphosphate

Le sel de sodium de l'adénosine 5'-triphosphate (ATP) a été obtenu chez SIGMA (Ref A3377, CAS# 56-65-5) dans l'eau Milli-Q^{®} en ajustant le pH entre 5 et 8 de sorte à obtenir une solution à 1120 mM en sel de sodium de l'adénosine 5'-triphosphate.

### EXEMPLE 2 - Effet de l'uridine sur la viscosité de différentes solutions de protéines thérapeutiques hautement concentrées comparativement à celui de l'arginine et du NaCl.

Cet exemple illustre comment l'uridine réduit la viscosité de trois formulations d'anticorps monoclonaux en comparaison des composés de référence que sont le chlorhydrate d'arginine et le NaCl.

Pour cet exemple, trois solutions d'anticorps monoclonaux i.e. une solution de bevacizumab (un IgG1 commercialisé sous le nom d'Avastin^{®} par Genentech / Hoffmann La Roche), une solution d'infliximab (un IgG1/κ commercialisé sous le nom de Remicade^{®} par Schering-Plough) et une solution de trastuzumab (un IgG1/κ commercialisé sous le nom d'Herceptin^{®} par Genentech), ont été concentrées par ultrafiltration sur des unités de filtration centrifuger Amicon^{®} Ultra-15 possédant un seuil de coupure de 50 kDa. La concentration en protéine de chaque solution concentrée a été mesurée par spectroscopie UV (absorbance à 280 nm) après dilution des échantillons.

La solution commerciale de bevacizumab (25 mg/mL) a été concentrée à 268 mg/mL. Pour infliximab et trastuzumab, les produits commerciaux sont des lyophilisats qui ont tout d'abord été reconstitués à l'aide d'eau pour injection à la concentration suggérée par le fabricant (i.e. 10 mg/mL pour infliximab et 21 mg/mL pour trastuzumab). Une fois reconstituées, les formulations de infliximab et de trastuzumab ont été concentrées à respectivement 183 mg/mL et 243 mg/mL.

Les pH des formulations commerciales de protéines thérapeutiques utilisées pour ces essais sont résumés dans le tableau II ci-dessous :

**Tableau II**

| | bevacizumab | infliximab | trastuzumab |
|---|---|---|---|
| pH de la formulation commerciale | 6,2 ± 0,2 | 7,2 ± 0,2 | 6,0 ± 0,2 |

L'effet de l'uridine comparativement à celui de l'arginine et du NaCl sur la viscosité de ces trois solutions de protéines thérapeutiques hautement concentrées a ensuite été évalué. Pour cela, la viscosité a été mesurée à l'aide d'un rhéomètre cône/plan (TA Instrument, AR 2000 Ex, géométrie de diamètre 20 mm, cône de 0,5131°, trappe à solvent remplie d'eau) à une température de 21°C et en mode Balayage (Flow Sweep) avec des taux de cisaillements compris entre 5000 et 50 s⁻¹ (3 points par décade - steady-state sensing). Toutes les valeurs de viscosité sont déterminées au niveau du plateau Newtonien.

Pour cet exemple, les formulations « Protéine + Urid », « Protéine + Arg » et « Protéine + NaCl » ont été préparées par mélange d'une des solutions d'uridine, d'arginine ou de NaCl préparées précédemment, avec de l'eau Milli-Q^{®} et avec une des solutions de protéine concentrée préparées précédemment de sorte à atteindre une concentration finale en uridine, arginine ou NaCl de 150 mmol/L (mM) et une concentration finale en protéine de 220 mg/mL pour bevacizumab, 150 mg/mL pour infliximab et de 200 mg/mL pour trastuzumab. Les échantillons ainsi préparés ont été stockés à température ambiante et ont été analysés à l'aide du rhéomètre dans les 24 heures suivant leur préparation.

Les solutions « témoins » sont préparées par dilution dans l'eau Milli-Q^{®}, de sorte à atteindre une concentration finale en protéine de 220 mg/mL pour bevacizumab, 150 mg/mL pour infliximab et de 200 mg/mL pour trastuzumab.

Le pH de ces formulations (« Protéine + Urid », « Protéine + Arg » et « Protéine + NaCl » et « témoins ») est le même que celui de la formulation commerciale de la protéine thérapeutique correspondante (Tableau II).

Les résultats des mesures relatives à ce deuxième exemple sont présentés dans le tableau III ci-après sous la forme de pourcentages de réduction de viscosité par rapport à la solution témoin de protéine thérapeutique seule, à la même concentration en protéine que les formulations « Protéine + Urid », « Protéine + Arg » ou « Protéine + NaCl ».

**Tableau III**

| Concentration testée | % de réduction de viscosité par rapport au contrôle « Protéine Seule » | | |
|---|---|---|---|
| | bevacizumab 220 mg/mL | infliximab trastuzumab 150 mg/mL 200 mg/mL | |
| 150 mM NaCl | 24% | 6% | 34% |
| 150 mM Arq | 46% | 55% | 48% |
| 150 mM Urid | 64% | 70% | 35% |

Les données présentées dans le tableau III démontrent qu'en présence de 150 mM d'uridine, la viscosité des 3 solutions de protéines hautement concentrées est significativement réduite, de 64% avec bevacizumab à 220 mg/mL, de 70% avec infliximab à 150 mg/mL et de 35% avec trastuzumab à 200 mg/L. En outre, l'uridine s'avère être significativement plus efficace que l'arginine et le NaCl pour abaisser la viscosité des solutions hautement concentrées de bevacizumab et d'infliximab.

### EXEMPLE 3 - Effet de la cytidine, de la 2'-deoxycytidine et de l'uridine sur la viscosité de deux formulations aqueuses d'anticorps monoclonaux hautement concentrées comparativement à celui de l'arginine, du NaCl et du sel de sodium de l'adénosine 5'-triphosphate.

Cet exemple illustre l'abaissement la viscosité de solutions hautement concentrées de bevacizumab et d'infliximab par la cytidine, la 2'-deoxycytidine et l'uridine en comparaison de composés de référence tels que l'arginine et le NaCl et en comparaison du sel de sodium de l'adénosine 5'-triphosphate.

Dans cette étude, les solutions de bevacizumab et d'infliximab hautement concentrées utilisées sont les mêmes que celles qui ont été préparées et utilisées précédemment. Les formulations « Protéine + Cyt », « Protéine + Deoxycyt », « Protéine + Urid », « Protéine + Arg », « Protéine + NaCl » et « Protéine + ATP » ont été préparées en présence de 150 mM de cytidine, de 2'-deoxycytidine, d'uridine, d'arginine, de NaCl ou de sel de sodium de l'adénosine 5'-triphosphate.

La viscosité des formulations « Protéine + Cyt », « Protéine + Deoxycyt », « Protéine + Urid » a ensuite été mesurée comme décrit à l'exemple 2 et comparée à celle obtenue pour les formulations « Protéine + Arg », « Protéine + NaCl » et « Protéine + ATP ». Le pH de ces formulations est le même que celui de la formulation de protéine thérapeutique correspondante, c'est-à-dire 6,2 ± 0,2 pour bevacizumab et de 7,2 ± 0,2 pour infliximab.

Les résultats des mesures relatives à cet exemple sont présentés dans les tableaux IV et V ci-après sous la forme de pourcentages de réduction de viscosité par rapport à la solution témoin de protéine thérapeutique seule, à la même concentration en protéine que les formulations « Protéine + Cyt », « Protéine + Deoxycyt », « Protéine + Urid », « Protéine + Arg », « Protéine + NaCl » et « Protéine + ATP ».

**Tableau IV**

| | Concentration testée | % de réduction de viscosité par rapport au contrôle « Protéine Seule » |
|---|---|---|
| bevacizumab 220 mg/mL | 150 mM NaCl | 24% |
| | 150 mM Arg | 46% |
| | 150 mM ATP | -14% |
| | 150 mM Cyt | 43% |
| | 150 mM Deoxycyt | 51% |
| | 150 mM Urid | 64% |

Les données présentées dans le tableau IV démontrent qu'utilisés à 150 mM, la cytidine, la 2'-deoxycytidine et l'uridine réduisent la viscosité de bevacizumab à 220 mg/mL de façon significativement plus efficace que le NaCl à la même concentration. Par ailleurs, la 2'-deoxycytidine et l'uridine réduisent la viscosité de bevacizumab à 220 mg/mL de façon plus efficace que l'arginine à la même concentration.

Le sel de sodium de l'adénosine 5'-triphosphate ne permet pas *a fortiori* de réduire la viscosité de bevacizumab. Le sel de sodium de l'adénosine 5'-triphosphate augmente au contraire la viscosité de ce dernier (+14% d'augmentation).

**Tableau V**

| | Concentration testée | % de réduction de viscosité par rapport au contrôle « Protéine Seule » |
|---|---|---|
| infliximab 150 mg/mL | 150 mM ATP | 0% |
| | 150 mM NaCl | 6% |
| | 150 mM Arg | 55% |
| | 150 mM Urid | 70% |
| | 150 mM Deoxycyt | 75% |
| | 150 mM Cyt | 76% |

Les données présentées dans le tableau V démontrent qu'utilisées à 150 mM, la cytidine, la 2'-deoxycytidine et l'uridine réduisent la viscosité de infliximab à 150 mg/mL de façon significativement plus efficace que le NaCl et l'arginine à la même concentration. La cytidine, la 2'-deoxycytidine et l'uridine sont notamment plus de 10 fois plus efficaces que le NaCl.

Le sel de sodium de l'adénosine 5'-triphosphate ne permet pas de réduire la viscosité de infliximab.

### EXEMPLE 4 - Effet de la concentration d'un anticorps monoclonal sur l'abaissement de viscosité en présence de cytidine comparativement à celui en présence d'arginine.

L'objectif de cet exemple est de comparer l'efficacité pour la réduction de la viscosité de la cytidine à celle de l'arginine en fonction de la concentration en anticorps.

La solution d'infliximab hautement concentrée utilisée est la même que celle qui a été préparée précédemment. Les formulations « Protéine + Cyt » et « Protéine + Arg » ont été préparées en présence de 150 mM de cytidine ou d'arginine et avec des concentrations décroissantes de 150 mg/mL à 80 mg/mL en infliximab.

Le pH de ces formulations est le même que celui de la formulation commerciale d'infliximab, c'est-à-dire 7,2 ± 0,2. La viscosité des formulations ainsi préparées a été mesurée comme décrit dans l'exemple 2.

Les résultats des mesures relatives à cet exemple sont présentés dans le tableau VI ci-après sous la forme de pourcentages de réduction de viscosité par rapport à la solution infliximab témoin.

**Tableau VI**

| | % de réduction de viscosité par rapport à la solution infliximab témoin | |
|---|---|---|
| Concentration en infliximab (mg/mL) | Arg à 150 mM | Cyt à 150 mM |
| 80 | 36% | 58% |
| 105 | 47% | 68% |
| 125 | 46% | 69% |
| 140 | 59% | 77% |
| 150 | 55% | 76% |

Les données présentées dans le tableau VI montrent que la cytidine abaisse la viscosité d'infliximab de façon significativement plus efficace que l'arginine à toutes les concentrations d'infliximab testées

### EXEMPLE 5 - Effet de la concentration en cytidine sur la diminution de viscosité d'une formulation aqueuse d'anticorps monoclonal hautement concentrée comparativement à celui de l'arginine et du NaCl.

La solution d'infliximab hautement concentrée utilisée est la même que celle qui a été préparée et utilisée précédemment. Les formulations « Protéine + Cyt », « Protéine + Arg » et « Protéine + NaCl » ont été préparées en présence de concentrations croissantes de 0 à 300 mM de cytidine, d'arginine ou de NaCl.

Le pH de ces formulations est le même que celui de la formulation commerciale d'infliximab, c'est-à-dire 7,2 ± 0,2. La viscosité des formulations ainsi préparées a été mesurée comme décrit dans l'exemple 2.

Les résultats des mesures relatives à cet exemple sont présentés dans le tableau VII ci-après sous la forme de pourcentages de réduction de viscosité par rapport à la solution témoin de protéine thérapeutique seule, à la même concentration en protéine que les formulations « Protéine + Cyt », « Protéine + Arg » et « Protéine + NaCl ».

**Tableau VII**

| | Concentration testée | Arg % de réduction de viscosité par rapport au contrôle « Protéine Seule » | Cyt % de réduction de viscosité par rapport au contrôle « Protéine Seule » | NaCt % de réduction de viscosité par rapport au contrôle « Protéine Seule » |
|---|---|---|---|---|
| infliximab | 50 mM | 32% | 39% | 20% |
| 105 | 150 mM | 46% | 68% | -6% |
| mg/mL | 300 mM | 58% | 72% | -90% |

Les données présentées dans le tableau VII montrent que quelle que soit la concentration en cytidine utilisée, cette dernière est toujours significativement plus efficace que l'arginine et le NaCl.

### EXEMPLE 6 - Exemples de compositions selon l'invention.

6.1 Composition sous forme de solution hautement concentrée de bevacizumab dont le pH est de 6,2 comprenant de la cytidine

| Ingrédient : | Concentration (mg/mL) : |
|---|---|
| bevacizumab | 200 |
| Phosphate de sodium (monobasique, monohydrate) | 5,8 |
| Phosphate de sodium (dibasique, anhydre) | 1,2 |
| Dihydrate d'α,α-trehalose | 60 |
| Polysorbate 20 | 0,4 |
| Cytidine (Cyt) | 36,5 |
| Eau pour injection, USP | qsp |

| | |
|---|---|
| qsp signifie quantité suffisante pour. | |

6.2 Composition sous forme de solution hautement concentrée de bevacizumab dont le pH est de 6,2 comprenant de l'uridine

| Ingrédient : | Concentration (mg/mL) : |
|---|---|
| bevacizumab | 200 |
| Phosphate de sodium (monobasique, monohydrate) | 5,8 |
| Phosphate de sodium (dibasique, anhydre) | 1,2 |
| Dihydrate d'α,α-trehalose | 60 |
| Polysorbate 20 | 0,4 |
| Uridine (Urid) | 36,6 |
| Eau pour injection, USP | qsp |

6.3 Composition sous forme de solution hautement concentrée d'infliximab dont le pH est de 7,2 comprenant de l'uridine

| Ingrédient: | Concentration (mg/mL) : |
|---|---|
| infliximab | 150 |
| Phosphate de sodium (monobasique, monohydrate) | 0,22 |
| Phosphate de sodium (dibasique, dihydrate) | 0,61 |
| Sucrose | 50 |
| Polysorbate 80 | 0,05 |
| Uridine (Urid) | 36,6 |
| Eau pour injection, USP | qsp |

6.4 Composition sous forme de solution hautement concentrée d'infliximab dont le pH est de 7,2 comprenant de la cytidine

| Ingrédient : | Concentration (mg/mL) : |
|---|---|
| infliximab | 150 |
| Phosphate de sodium (monobasique, monohydrate) | 0,22 |
| Phosphate de sodium (dibasique, dihydrate) | 0,61 |
| Sucrose | 50 |
| Polysorbate 80 | 0,05 |
| Cytidine (Cyt) | 36,5 |
| Eau pour injection, USP | qsp |

6.5 Composition sous forme de solution hautement concentrée de bevacizumab dont le pH est de 6,2 comprenant de la 2'-deoxycytidine

| Ingrédient : | Concentration (mg/mL) : |
|---|---|
| bevacizumab | 200 |
| Phosphate de sodium (monobasique, monohydrate) | 5,8 |
| Phosphate de sodium (dibasique, anhydre) | 1,2 |
| Dihydrate d'α,α-trehalose | 60 |
| Polysorbate 20 | 0,4 |
| 2'-Deoxycytidine (Deoxycyt) | 34,1 |
| Eau pour injection, USP | qsp |

6.6 Composition sous forme de solution hautement concentrée d'infliximab dont le pH est de 7,2 comprenant de la 2'-deoxycytidine

| Ingrédient : | Concentration (mg/mL) : |
|---|---|
| infliximab | 150 |
| Phosphate de sodium (monobasique, monohydrate) | 0,22 |
| Phosphate de sodium (dibasique, dihydrate) | 0,61 |
| Sucrose | 50 |
| Polysorbate 80 | 0,05 |
| 2'-Deoxycytidine (Deoxycyt) | 34,1 |
| Eau pour injection, USP | qsp |

6.7 Composition sous forme de solution hautement concentrée de bevacizumab dont le pH est de 6,2 comprenant de la thymidine

| Ingrédient : | Concentration (mg/mL) : |
|---|---|
| bevacizumab | 200 |
| Phosphate de sodium (monobasique, monohydrate) | 5,8 |
| Phosphate de sodium (dibasique, anhydre) | 1,2 |
| Dihydrate d'α,α-trehalose | 60 |
| Polysorbate 20 | 0,4 |
| Thymidine (Thym) | 36,3 |
| Eau pour injection, USP | qsp |

6.8 Composition sous forme de solution hautement concentrée d'infliximab dont le pH est de 7,2 comprenant de la thymidine

| Ingrédient : | Concentration (mg/ml) : |
|---|---|
| infliximab | 150 |
| Phosphate de sodium (monobasique, monohydrate) | 0,22 |
| Phosphate de sodium (dibasique, dihydrate) | 0,61 |
| Sucrose | 50 |
| Polysorbate 80 | 0.05 |
| Thymidine (Thym) | 36,3 |
| Eau pour injection, USP | qsp |

6.9 Composition sous forme de solution hautement concentrée de bevacizumab dont le pH est de 6,2 comprenant de la ribothymidine

| Ingrédient : | Concentration (mg/mL) : |
|---|---|
| bevacizumab | 200 |
| Phosphate de sodium (monobasique, | 5,8 |
| monohydrate) | |
| Phosphate de sodium (dibasiaue, | 1.2 |
| anhydre) | |
| Dihydrate d' α,α-trehalose | 60 |
| Polysorbate 20 | 0,4 |
| Ribothymidine | 38.7 |
| Eau pour injection USP | -qs-2 |

6.10 Composition sous forme de solution hautement concentrée de bevacizumab dont le pH est de 6,2 comprenant de la 2'-deoxyuridine

| Ingrédient : | Concentration (mg/mL) : |
|---|---|
| bevacizumab, | 200 |
| Phosphate de sodium (monobasique, | 5,8 |
| monohydrate). | |
| Phosphate de sodium (dibasique, | 1.2 |
| anhydre) | |
| Dihydrate d'α,α-trehalose | 60 |
| Polysorbate 20 | 0,4 |
| 2'-deoxyuridine | 34.2 |
| Eau pour injection, USP | qsp |

6.11 Composition sous forme de solution hautement concentrée d'infliximab dont le pH est de 7,2 comprenant de la ribothymidine

| Ingrédient : | Concentration (mg/mL) : |
|---|---|
| infliximab | 150 |
| Phosphate de sodium (monobasique monohydrate) | 0.22 |
| Phosphate de sodium (dibasique, dihydrate) | 0,61 |
| Sucrose | 50 |
| Polvsorbate 80 | 0,05 |
| Ribothymidine | 38.7 |
| Eau pour injection USP | qsp |

6.12 Composition sous forme de solution hautement concentrée d'infliximab dont le pH est de 7,2 comprenant de la 2'-deoxyuridine

| Ingrédient : | Concentration (mg/mL) : |
|---|---|
| infliximab | 150 |
| Phosphate de sodium (monobasique monohydrate) | 0,22 |
| Phosphate de sodium (dibasique, dihydrate) | 0,61 |
| Sucrose | 50 |
| polysorbate 80 | 0,05 |
| 2'-deoxyuridine | 34,2 |
| Eau pour injection, USP | qsp |

## Revendications

1. Composition comprenant, en solution aqueuse, au moins une protéine comportant au moins un fragment d'anticorps, et au moins un agent réducteur de viscosité soluble dans l'eau, choisi dans le groupe constitué par la cytidine, la 2'-deoxycytidine, l'uridine, la 2'-deoxyuridine, la thymidine, la ribothymidine , seule ou en mélange.

2. Composition selon la revendication 1, **caractérisée en ce que** l'agent réducteur de viscosité est choisi dans le groupe constitué de la cytidine, la 2'-deoxycytidine et l'uridine seule ou en mélange.

3. Composition selon l'une quelconque des revendications 1 ou 2, **caractérisée en ce que** la concentration en protéine comportant au moins un fragment d'anticorps dans la formulation finale est supérieure ou égale à 50 mg/mL.

4. Composition selon l'une quelconque des revendications précédentes, **caractérisée en ce que** son pH est compris entre 5 et 8.

5. Composition selon l'une quelconque des revendications précédentes, **caractérisée en ce que** la concentration en agent réducteur de viscosité est supérieure ou égale à 10 mM.

6. Composition selon l'une quelconque des revendications précédentes, **caractérisée en ce que** la protéine comportant au moins un fragment d'anticorps est choisie parmi les anticorps monoclonaux, les anticorps polyclonaux, les protéines de fusion, les nanobodies, les anticorps bispécifiques, les anticorps couplés à des principes actifs cytotoxiques (ADC).

7. Composition selon l'une quelconque des revendications précédentes, **caractérisée en ce qu'**elle est une solution aqueuse comprenant :
- au moins un tampon,
- au moins un tensioactif, et
- au moins un diluant.

8. Utilisation d'au moins un agent réducteur de viscosité soluble dans l'eau, choisi dans le groupe constitué par la cytidine, la 2'-deoxycytidine, l'uridine, la 2'-deoxyuridine, la thymidine, la ribothymidine , seule ou en mélange à une concentration dans la formulation finale supérieure ou égale à 10 mM pour abaisser la viscosité d'une solution aqueuse difficilement injectable d'au moins une protéine comportant au moins un fragment d'anticorps dont la concentration est supérieure ou égale à 50 mg/mL et dont le pH est compris entre 5 et 8, d'une valeur d'au moins 10 % par rapport à la viscosité d'une solution aqueuse difficilement injectable d'au moins une protéine comportant au moins un fragment d'anticorps de même concentration et de même pH ne contenant pas ledit agent réducteur de viscosité.

9. Utilisation selon la revendication 8, **caractérisée en ce que** la concentration en protéine comportant au moins un fragment d'anticorps dans la formulation finale est comprise entre 50 et 350 mg/mL.

10. Utilisation selon l'une quelconque des revendications 8 à 9, **caractérisée en ce que** la concentration finale en agent réducteur de viscosité est comprise entre 10 et 350 mM.

11. Utilisation selon l'une quelconque des revendications 8 à 10, **caractérisée en ce que** l'agent réducteur de viscosité est choisi dans le groupe constitué par la cytidine, la 2'-deoxycytidine, l'uridine et leurs mélanges.

12. Procédé d'abaissement de la viscosité d'une solution aqueuse difficilement injectable d'au moins une protéine comportant au moins un fragment d'anticorps dont la concentration est supérieure ou égale à 50 mg/mL et dont le pH est compris entre 5 et 8, d'une valeur d'au moins 10% par rapport à la viscosité d'une solution aqueuse d'au moins une protéine comportant au moins un fragment d'anticorps de même concentration et de même pH ne contenant pas ledit agent réducteur de viscosité consistant à préparer une solution aqueuse comprenant :
- ladite protéine d'intérêt comportant au moins un fragment d'anticorps à une concentration supérieure ou égale à 50 mg/mL, et au moins
- un agent réducteur de viscosité soluble dans l'eau, choisi dans le groupe constitué par la cytidine, la 2'-deoxycytidine, l'uridine, la 2'-deoxyuridine, la thymidine et la ribothymidine , seule ou en mélange.

13. Composition pharmaceutique pour l'administration par voie parentérale telle que l'injection intraveineuse (IV), comprenant en solution aqueuse, au moins une protéine comportant au moins un fragment d'anticorps, et au moins un agent réducteur de viscosité choisi dans le groupe constitué par la cytidine, la 2'-deoxycytidine, l'uridine, la 2'-deoxyuridine, la thymidine, la ribothymidine , seule ou en mélange.

14. Composition pharmaceutique pour l'administration par voie sous-cutanée (SC) comprenant en solution aqueuse, au moins une protéine comportant au moins un fragment d'anticorps, et au moins un agent réducteur de viscosité choisi dans le groupe constitué par la cytidine, la 2'-deoxycytidine, l'uridine, la 2'-deoxyuridine, la thymidine, la ribothymidine , seule ou en mélange.

15. Composition pharmaceutique pour l'administration par voie intramusculaire (IM) comprenant en solution aqueuse, au moins une protéine comportant au moins un fragment d'anticorps, et au moins un agent réducteur de viscosité choisi dans le groupe constitué par la cytidine, la 2'-deoxycytidine, l'uridine, la 2'-deoxyuridine, la thymidine, la ribothymidine, seule ou en mélange.

## Patentansprüche

1. Zusammensetzung umfassend, in wässriger Lösung, wenigstens ein ein Antikörperfragment umfassendes Protein und wenigstens ein wasserlösliches, Viskosität verringerndes Agens, das ausgewählt ist aus der Gruppe bestehend aus Cytidin, 2'-Deoxycytidin, Uridin, 2-Deoxyuridin, Thymidin und Ribothymidin, alleine oder als eine Mischung.

2. Zusammensetzung nach Anspruch 1, **dadurch gekennzeichnet, dass** das Viskosität verringernde Agens ausgewählt ist aus der Gruppe bestehend aus Cytidin, 2'-Deoxycytidin und Uridin, alleine oder als eine Mischung.

3. Zusammensetzung nach einem der Ansprüche 1 oder 2, **dadurch gekennzeichnet, dass** die Konzentration des ein Antikörperfragment umfassenden Proteins in der Endformulierung mehr als oder gleich 50 mg/ml beträgt.

4. Zusammensetzung nach einem der vorhergehenden Ansprüche, **dadurch gekennzeichnet, dass** ihr pH zwischen 5 und 8 beträgt.

5. Zusammensetzung nach einem der vorhergehenden Ansprüche, **dadurch gekennzeichnet, dass** die Konzentration des Viskosität verringernden Agens mehr als oder gleich 10 mM beträgt.

6. Zusammensetzung nach einem der vorhergehenden Ansprüche, **dadurch gekennzeichnet, dass** das wenigstens ein Antikörperfragment umfassende Protein ausgewählt ist aus monoklonalen Antikörpern, polyklonalen Antikörpern, Fusionsproteinen, Nanobodies, bispezifischen Antikörpern, und mit zytotoxischen Wirksubstanzen gekoppelten Antikörpern.

7. Zusammensetzung nach einem der vorhergehenden Ansprüche, **dadurch gekennzeichnet, dass** sie eine wässrige Lösung ist umfassend:
- wenigstens einen Puffer,
- wenigstens ein oberflächenaktives Mittel, und
- wenigstens ein Verdünnungsmittel.

8. Verwendung wenigstens eines in Wasser löslichen, Viskosität verringernden Agens, das ausgewählt ist aus der Gruppe bestehend aus Cytidin, 2'-Deoxycytidin, Uridin, 2-Deoxyuridin, Thymidin und Ribothymidin, alleine oder als eine Mischung, in einer Konzentration in der Endformulierung von mehr als oder gleich 10 mM zum Verringern der Viskosität einer wässrigen, schwer injizierbaren Lösung von wenigstens einem, ein Antikörperfragment umfassenden Protein, dessen Konzentration mehr als oder gleich 50 mg/ml beträgt, und deren pH zwischen 5 und 8 beträgt, um einen Wert von wenigstens 10% bezogen auf die Viskosität einer wässrigen, schwer injizierbaren Lösung von wenigstens einem, wenigstens ein Antikörperfragment umfassenden Protein mit der gleichen Konzentration und mit dem gleichen pH, die nicht das Viskosität verringernde Agens enthält.

9. Verwendung nach Anspruch 8, **dadurch gekennzeichnet, dass** die Konzentration an Protein, das wenigstens ein Antikörperfragment umfasst, in der Endformulierung zwischen 50 und 350 mg/ml beträgt.

10. Verwendung nach einem der Ansprüche 8 und 9, **dadurch gekennzeichnet, dass** die Endkonzentration des Viskosität verringernden Agens zwischen 10 und 350 mM beträgt.

11. Verwendung nach einem der Ansprüche 8 bis 10, **dadurch gekennzeichnet, dass** das Viskosität verringernde Agens ausgewählt ist aus der Gruppe bestehend aus Cytidin, 2'-Deoxycytidin, Uridin und Mischungen davon.

12. Verfahren zum Verringern der Viskosität einer wässrigen, schwer injizierbaren Lösung aus wenigstens einem, ein Antikörperfragment umfassenden Protein, dessen Konzentration mehr als oder gleich 50 mg/ml beträgt, und deren pH zwischen 5 und 8 beträgt, um einen Wert von wenigstens 10 % bezogen auf die Viskosität einer wässrigen Lösung von wenigstens einem, wenigstens ein Antikörperfragment umfassenden Protein mit der gleichen Konzentration und dem gleichen pH, die nicht das Viskosität verringernde Agens enthält, umfassend Herstellen einer wässrigen Lösung umfassend:
- das interessierende Protein umfassend wenigstens ein Antikörperfragment mit einer Konzentration von mehr als oder gleich 50 mg/ml, und wenigstens
- ein in Wasser lösliches, Viskosität verringerndes Agens, das ausgewählt ist aus der Gruppe bestehend aus Cytidin, 2'-Deoxycytidin, Uridin, 2'-Deoxyuridin, Thymidin und Ribothymidin, alleine oder als eine Mischung.

13. Pharmazeutische Zusammensetzung zur parenteralen Verabreichung wie intravenöser (IV) Injektion, umfassend in wässriger Lösung wenigstens ein, wenigstens ein Antikörperfragment umfassendes Protein und wenigstens ein Viskosität verringerndes Agens, das ausgewählt ist aus der Gruppe bestehend aus Cytidin, 2'-Deoxycytidin, Uridin, 2'-Deoxyuridin, Thymidin und Ribothymidin, alleine oder als eine Mischung.

14. Pharmazeutische Zusammensetzung zur subkutanen (SC) Verabreichung, umfassend in wässriger Lösung wenigstens ein, wenigstens ein Antikörperfragment umfassendes Protein und wenigstens ein Viskosität verringerndes Agens, das ausgewählt ist aus der Gruppe bestehend aus Cytidin, 2'-Deoxycytidin, Uridin, 2'-Deoxyuridin, Thymidin und Ribothymidin, alleine oder als eine Mischung.

15. Pharmazeutische Zusammensetzung zur intramuskulären (IM) Verabreichung, umfassend in wässriger Lösung wenigstens ein, wenigstens ein Antikörperfragment umfassendes Protein und wenigstens ein Viskosität verringerndes Agens, das ausgewählt ist aus der Gruppe bestehend aus Cytidin, 2'-Deoxycytidin, Uridin, 2'-Deoxyuridin, Thymidin und Ribothymidin, alleine oder als eine Mischung.

## Claims

1. Composition comprising, in aqueous solution, at least one protein comprising at least one antibody fragment, and at least one viscosity-reducing agent soluble in water, chosen from the group consisting of cytidine, 2'-deoxycytidine, uridine, 2'-deoxyuridine, thymidine, ribothymidine, alone or as a mixture.

2. Composition according to claim 1, **characterized in that** the viscosity-reducing agent is chosen from the group consisting of cytidine, 2'-deoxycytidine, and uridine, alone or as a mixture.

3. Composition according to any one of claims 1 or 2, **characterized in that** the concentration of protein comprising at least one antibody fragment in the final formulation is greater than or equal to 50 mg/mL.

4. Composition according to any one of the preceding claims, **characterized in that** its pH is between 5 and 8.

5. Composition according to any one of the preceding claims, **characterized in that** the concentration of viscosity-reducing agent is greater than or equal to 10 mM.

6. Composition according to any one of the preceding claims, **characterized in that** the protein comprising at least one antibody fragment is chosen from among the monoclonal antibodies, the polyclonal antibodies, the fusion proteins, the nanobodies, the bispecific antibodies, and the antibodies coupled to active cytotoxic principles (ADC).

7. Composition according to any one of the preceding claims, **characterized in that** it is an aqueous solution comprising:
- at least one buffer,
- at least one surfactant, and
- at least one diluent.

8. Use of at least one viscosity-reducing agent soluble in water, chosen from the group consisting of cytidine, 2'-deoxycytidine, uridine, 2'-deoxyuridine, thymidine, ribothymidine, alone or in mixture at a concentration in the final formulation equal to or greater than 10 mM to lower the viscosity of a hard to inject aqueous solution of at least one protein comprising at least one antibody fragment whose concentration is greater than or equal to 50 mg/mL, and whose pH is between 5 and 8, by a value of at least 10% compared to the viscosity of a hard to inject aqueous solution of at least one protein comprising at least one antibody fragment of the same concentration and the same pH, not containing said viscosity-reducing agent.

9. Use according to claim 8, **characterized in that** the concentration of protein comprising at least one antibody fragment in the final formulation is between 50 and 350 mg/mL.

10. Use according to any one of claims 8 to 9, **characterized in that** the final concentration of viscosity-reducing agent is between 10 and 350 mM.

11. Use according to any one of claims 8 to 10, **characterized in that** the viscosity-reducing agent is chosen from the group consisting of cytidine, 2'-deoxycytidine, and uridine, and their mixtures.

12. Process for lowering the viscosity of a hard to inject aqueous solution of at least one protein comprising at least one antibody fragment whose concentration is greater than or equal to 50 mg/mL, and whose pH is between 5 and 8, by a value of at least 10% compared to the viscosity of a hard to inject aqueous solution of at least one protein having at least one antibody fragment of the same concentration and the same pH, not containing said viscosity-reducing agent, consisting in the preparing of an aqueous solution comprising:
- said protein of interest comprising at least one antibody fragment at a concentration greater than or equal to 50 mg/mL, and at least
- one viscosity-reducing agent soluble in water, chosen from the group consisting of cytidine, 2'-deoxycytidine, uridine, 2'-deoxyuridine, thymidine, ribothymidine, alone or as a mixture.

13. Pharmaceutical composition for administration by parenteral route, such as intravenous (IV) injection, comprising in aqueous solution at least one protein comprising at least one antibody fragment, and at least one viscosity-reducing agent chosen from the group consisting of cytidine, 2'-deoxycytidine, uridine, 2'-deoxyuridine, thymidine, ribothymidine, alone or as a mixture.

14. Pharmaceutical composition for administration by subcutaneous (SC) route, comprising in aqueous solution at least one protein comprising at least one antibody fragment, and at least one viscosity-reducing agent chosen from the group consisting of cytidine, 2'-deoxycytidine, uridine, 2'-deoxyuridine, thymidine, ribothymidine, alone or as a mixture.

15. Pharmaceutical composition for administration by intramuscular (IM) route, comprising in aqueous solution at least one protein having at least one antibody fragment, and at least one viscosity-reducing agent chosen from the group consisting of cytidine, 2'-deoxycytidine, uridine, 2'-deoxyuridine, thymidine, ribothymidine, alone or as a mixture.
